# EUROPÄISCHE PATENTSCHRIFT

(11) **EP 2 796 520 B1**
(45) Veröffentlichungstag und Bekanntmachung des Hinweises auf die Patenterteilung: **30.05.2018**
(21) Anmeldenummer: 14163381.8
(22) Anmeldetag: 03.04.2014
(51) Int. Cl.: C09D 183/08, C03C 17/30, C09D 183/12

(54) **BEHÄLTER MIT GERINGER PARTIKELEMISSION UND REIBKONTROLLIERTER TROCKENGLEITOBERFLÄCHE, SOWIE VERFAHREN ZU DESSEN HERSTELLUNG**
CONTAINERS WITH LOW PARTICLE EMISSION AND FRICTION-CONTROLLED DRY SLIDING SURFACE, AND METHOD FOR THE PRODUCTION THEREOF
RÉCIPIENT AYANT DE FAIBLES ÉMISSIONS DE PARTICULES ET UNE SURFACE LISSE ET SÈCHE À FRICTION CONTRÔLÉE ET SON PROCÉDÉ DE FABRICATION

(30) Priorität: 11.04.2013 DE 102013103676
(43) Veröffentlichungstag der Anmeldung: 29.10.2014
(73) Patentinhaber: Schott AG, 55122 Mainz (DE)
(72) Erfinder: Bicker, Matthias, 55126 Mainz (DE); Henze, Inka, 55268 Nieder-Olm (DE); Schuhmacher, Jörg, 70806 Kornwestheim (DE); Riethmueller, Franziska, 60326 Frankfurt am Main (DE); Hormes, Robert, 9403 Goldach (CH); Helbig, Christian, 9014 St. Gallen (CH); Walther, Marten, 31061 Alfeld (DE); Lohmeyer, Manfred, 55299 Nackenheim (DE)
(74) Vertreter: Blumbach · Zinngrebe Patent- und Rechtsanwälte

(56) Entgegenhaltungen:
- EP-A1- 1 391 249
- US-A1- 2009 087 646

## Beschreibung

Die Erfindung bezieht sich allgemein auf Behälter und insbesondere auf Behälter als Teil eines Pharmapackmittels oder einer medizinischen Vorrichtung oder einer sterilen Verpackung, wie zum Beispiel Spritzen-, Karpulen- oder Kanülensysteme sowie Pharmafläschchen (Vials).

An Pharmapackmittel wie Spritzen-, Karpulen- oder Kanülensysteme sowie Pharmafläschchen werden hohe Anforderungen an die Reibungseigenschaften der Innenseite der Packmittel gestellt. So sollen der Spritzenkolben oder der Stopfen eines Fläschchens mit möglichst geringer Reibung über die Innenseite der Spritze oder des Fläschchens gleiten. Gleichzeitig dürfen möglichst wenig Partikel oder Partikel-bildende Substanzen oder migrierbare Gleitöle von der Oberfläche der Innenseite des Packmittels in den pharmazeutischen Inhalt, einen pharmazeutischen Wirkstoff, gelangen, um eine Kontamination des Inhalts oder eine unerwünschte Wechselwirkung von Partikeln mit den Wirkstoff-Molekülen oder anderen Bestandteilen des Inhalts zu vermeiden. Beispielsweise sind Silikonöl-basierte Partikel als potentielle Trigger von Protein-Aggregation bekannt. Insbesondere proteinbasierte Wirkstoffformulierungen können sehr sensitiv bezüglich Kontaminationen und Wechselwirkungen mit Partikeln reagieren. Dieser Begriff bezieht sich auf alle flüssigen Lösungen, die Biomoleküle enthalten. Beispielsweise kann es sich dabei um wässrige oder alkoholische Formulierungen handeln. Die in der Lösung enthaltenen Biomoleküle können Peptide, Protein-Fragmente, Proteine, wie speziell besondere Spezies von Proteinen, wie monoklonale Antikörper, polyklonale Antikörper, Liganden, Rezeptoren, Antigene, Enzyme, die natürlich oder rekombinant hergestellt wurden als auch Derivate von diesen Biomolekülen sein.

Ferner sollen Eigenschaften hinsichtlich Reibung und Partikelemission auch über eine längere Lagerungsdauer erhalten bleiben.

Zur Erreichung einer Silikon-freien Spritze wurde bereits vorgeschlagen, die Innenseite des Packmittels mit einem alternativen Gleitöl bestehend aus einer fluorierten chemischen Verbindung zu beschichten. So wird beispielweise in der US 8 124 207 B2 vorgeschlagen, auf der Oberfläche eines Pharmaartikels Gleitöl aus einem Perfluorpolyether (PFPE) oder einem funktionalisierten Perfluorpolyether aufzutragen. Die mit diesem Gleitölversehene Oberfläche wird einem Flammen-Plasma bzw. einem Atmosphären-Plasma oder ionisierender Strahlung bzw. einer Energiequelle bei Atmosphärendruck ausgesetzt, wodurch, gemäß der Lehre dieser Patentschrift erst die gewünschten Gleiteigenschaften erzielt werden. Die gewünschten Gleiteigenschaften werden also erst durch ein sehr aufwändiges Verfahren unter Verwendung von mehrstufigen Prozessschritten erreicht. Ferner handelt es sich dabei um keine Öl-freie Lösung sondern das Silikonöl wird lediglich gegen ein anderes Gleitöl ersetzt. Dieses Perfluorpolyether-basierte Gleitöl enthält auch nach einer Plasma-Vernetzung freies Gleitöl, dass in die Medikamentenlösung migrieren kann und dort zu unerwünschten Nebenwirkungen führen kann. Ferner können auch in Verbindung mit diesem Verfahren und dem Gleitöl Partikel gebildet werden.

Weiterhin ist in der US 6 183 872 B1 und der WO 2011/060047 A1 die Beschichtung von Oberflächen mit fluorierten chemischen Verbindungen beschrieben worden, um Antireflex- und schmutzabweisende Eigenschaften zu erzielen. Als mögliche Einsatzgebiete wird unter anderem die Beschichtung optischer Elemente (Linsen, Displays etc.) genannt.

In der US 2011/0313363 A1 wird ein medizinischer Artikel beschrieben, der zunächst mit einer organopolysiloxanbasierten Schicht versehen wird. Durch plasmaunterstützte CVD wird eine zweite Schicht aufgebracht, die eine der folgenden Monomergruppen enthält: N-Vinylparrolidone, Vinylacetat, Ethylenoxid, Alkylacrylat, Alkylmethacrylat, Acrylamid, Acrylsäure sowie deren Mischungen. Diese Schichten reduzieren die Losbrechkraft und die Anzahl der emittierten Partikel lediglich um etwa einen Faktor 13,4 im Vergleich zu Silikon. Auch wird in der US 2011/0313363 A1 die Oberfläche des Pharmapackmittels mit Silikonölen versehen, um die Reibungskraft zu reduzieren. Dies hat aber den Nachteil, dass Silikonölmoleküle aus der Oberfläche in die im Packmittel aufbewahrten Wirkstofflösung migrieren können. Denn trotz Überbeschichtung des Silikons mit einer zweiten polymeren Schicht kann Silikonöl unter mechanischer oder thermischer Belastung aus dem Schichtverbund ausgelöst werden und ins Produkt migrieren.

Daher ist es eine wichtige Aufgabe der Erfindung, einen Behälter bereit zu stellen, der eine gegenüber dem Stand der Technik verringerte Partikelemission von der Oberfläche der Innenseite des Behälters in den Inhalt des Behälters aufweist.

Eine weitere Aufgabe der vorliegenden Erfindung ist es, einen Behälter bereit zu stellen mit in Kombination zur minimierten Partikel-Emission zusätzlich vorhandenen verbesserten Eigenschaften der Reibung an der Innenseite, der ohne hohen Aufwand beim Herstellungsprozess auskommt.

Eine weitere wichtige Aufgabe der vorliegenden Erfindung ist es, einen Behälter bereit zu stellen, der im Wesentlichen ohne oder sogar gänzlich ohne zusätzliche Gleitöle wie Silikonöle auskommt.

Auch soll ein Behälter bereit gestellt werden, der eine reibkontrollierte, lagerstabile Oberfläche aufweist, die in Wechselwirkung mit der Medikamentenlösung stabil ist.

Es ist auch eine Aufgabe der vorliegenden Erfindung, einen Behälter bereit zu stellen, dessen Eigenschaften hinsichtlich Reibung und Partikelemission auch über eine längere Lagerungsdauer erhalten bleiben.

Diese Aufgaben werden durch einen Behälter und ein Verfahren gemäß den unabhängigen Ansprüchen gelöst. Vorteilhafte Ausgestaltungen und Weiterbildungen sind in den jeweiligen abhängigen Ansprüchen angegeben.

Erfindungsgemäß wird ein Behälter mit einem Behälterkörper mit Außen- und einer Innenseite bereit gestellt, wobei die Innenseite Siliziumoxid-haltig ist und die Siliziumoxid-haltige Innenseite zumindest teilweise mit einer fluorhaltigen Verbindung modifiziert ist, wobei die fluorhaltige Verbindung über zumindest eine Si-O-Si-Bindung an das Siliziumoxid des Behälterkörpers chemisch gebunden ist. Die chemische Bindung kann dabei insbesondere auch eine chemisch kovalente Bindung sein.

In einer bevorzugten Ausführungsform ist der Behälter Bestandteil eines Pharmapackmittels oder einer medizinischen Vorrichtung oder einer sterilen Verpackung zur Aufbewahrung eines Produktes oder einer sterilen Verpackung zur Aufbewahrung eines pharmazeutischen Produktes.

Die fluorhaltige Verbindung ist hierbei eine Alkoxysilan-Verbindung mit folgendem Aufbau: 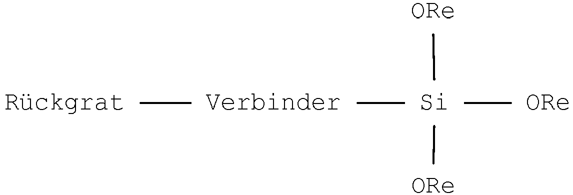 "ORe" steht für einen organischen Rest in Form einer Alkoxy-Gruppe. Die als "Rückgrat" bezeichnete Einheit der Verbindung ist fluorhaltig.

Der Verbinder, beziehungsweise die Verbinder-Einheit erlaubt es, dass die Moleküle der fluorhaltigen Verbindungen untereinander Bindungen eingehen, also Querverbindungen ausbilden. Dies erhöht die Stabilität der Verbindung und verringert wesentlich die Partikelemission aus der modifizierten Oberfläche der Innenseite des erfindungsgemäßen Pharmapackmittels.

Erfindungsgemäß weist die Alkoxysilan-Verbindung eines oder mehrere der folgenden Merkmale auf:
- Die Alkoxysilan-Verbindung enthält als Rückgrat ein Perfluorpolyether,
- das Rückgrat umfasst zumindest eine (CF₂)₃-Kette,
- das Rückgrat umfasst mehrere (CF₂)ₓ-Einheiten, wobei für alle (CF₂)ₓ-Einheiten x < 8 gilt,
- das Rückgrat enthält [(CF₂)ₓO]ₙ mit 3 < n < 1000, bevorzugt 4 < n < 200, besonders bevorzugt 5 < n < 100,
- das Rückgrat enthält als weitere Verzweigungen lineare und/oder verzweigte und/oder zyklische Strukturen,
- die Alkoxysilan-Verbindung enthält wenigstens eine CF₃ - Endgruppe,

Zur Sicherstellung der geringen Partikelemission weist die mit einer fluorhaltigen organischen Verbindung zumindest teilweise modifizierte Siliziumoxid-haltige Innenseite des Behälters eine Oberflächendichte unterhalb von 2.000 Partikeln/cm² für alle Partikel mit Durchmessern ≥ 2 µm auf. Alternativ oder zusätzlich gehen aus der Oberfläche der mit einer fluorhaltigen organischen Verbindung zumindest teilweise modifizierten Siliziumoxid-haltigen Innenseite gehen im Kontakt mit einer wässrigen Lösung pro ml Lösungsvolumen weniger als 10.000 Partikel mit einem Durchmesser ≥ 2 µm in die wässrige Lösung über-.

Der Behälter kann ferner einen Elastomerstopfen aufweisen, der in Reibpaarung mit der Innenseite des Behälters steht. In dieser Ausformung ist der Behälter beispielsweise eine Spritze oder eine Pharmakarpule.

Die modifizierte Innenseite des Behälters weist eines oder mehrere der folgenden Merkmale auf:
- der Kontaktwinkel für Wasser beträgt mehr als 100°, bevorzugt mehr als 105°, besonders bevorzugt mehr als 110°,
- der dynamische Kontaktwinkel beträgt beim Eintauchen mehr als 110° und im Rückzug mehr als 90°, vorzugsweise beim Eintauchen mehr als 115° und im Rückzug mehr als 105°,
- der Abrollwinkel liegt im Bereich von 1° bis 30°, bevorzugt im Bereich von 5° bis 20°, gemessen für ein Tröpfchen von 60 µl,
- die Oberfläche der Innenseite ist oleophob und / oder proteinabweisend,
- die Oberfläche der Innenseite ist oleophob und hydrophob.

In einer weiteren Ausführungsform umfasst der modifizierte Behälter ein Spritzen- oder Karpulensystem bestehend aus einem Kunststoffkörper aus Cycloolefin Polymer (COP) oder Cycloolefincopolymer (COC) und einer glasartigen Innenbeschichtung, beispielsweise einer Siliziumoxid-haltigen Zwischenschicht, an die die Fluoralkoxysilan-Verbindung zumindest teilweise unter Ausbildung einer Si-O-Si-Bindung chemisch angebunden ist. In einer besonderen weiteren Ausführungsform umfasst die Beschichtung eine weitere Haftvermittlerschicht, die direkt an das Polymersubstrat des Spritzenkörpers angekoppelt ist.

Ein weiteres Merkmal des Behälters ist, dass die reibmindernden Eigenschaften der mit einer fluorhaltigen Verbindung modifizierten Siliziumoxid-haltigen Innenseite auch nach einer beschleunigten Lagerung in Wasser bzw. Phosphatpuffer mit einem pH-Wert von 7 bei Lagerbedingungen von 40°C und 28 Tagen bestehen bleiben.

Ferner weist der Behälter wenigstens eine der folgenden Material- oder Substrat-Eigenschaften auf:
- der Behälter ist aus Glas der hydrolytischen Klasse 1 oder 2 gefertigt,
- der Behälter ist aus Borosilikat-Glas gefertigt,
- der Behälter ist ein Glaskörper mit Partikel-armer Oberfläche unterhalb von 2.000 Partikeln/cm² für alle Bor- oder Wolfram- oder Silizium-haltigen Partikel mit Durchmessern ≥ 2 µm,
- der Behälter ist aus Cyclo Olefin Polymer (COP) oder Cyclo Olefin Copolymer (COC) gefertigt,
- der Behälter ist ein Kunststoffkörper mit Partikel-armer Oberfläche unterhalb von 2.000 Partikeln/cm² für alle Partikel mit Durchmessern ≥ 2 µm,
- der Behälter ist ein Behälterkörper in Form eines Spritzenkörpers, Karpulenkörpers oder Fläschchens für medizinische Zwecke

In einer weiteren Ausführungsform der Erfindung weist der Behälter einen Behälterkörper auf, der nur auf einer Teiloberfläche O₁ mit einer fluorhaltigen Verbindung modifiziert ist, mit wenigstens einem der folgenden Merkmale:
- der Behälter ist auf wenigstens einer weiteren Teiloberfläche O₂ nicht mit einer fluorhaltigen Verbindung modifiziert,
- der Behälter ist auf zwei weiteren, räumlich getrennten Teiloberflächen O₁ und O₂ nicht mit einer fluorhaltigen Verbindung modifiziert,
- im Bereich von wenigstens einer nicht modifizierten Teiloberfläche ist der Behälter mit einem anderen Werkstoff verbunden,
- im Bereich von wenigstens einer nicht modifizierten Teiloberfläche ist ein Klebematerial aufgebracht.

Beispielsweise kann das Klebematerial aus einem Kleber bestehen, beispielsweise aus einem Klebstoff für medizinische Anwendungen, beispielsweise aus einem mittels elektromagnetischer Strahlung vernetzbarem Klebstoff. Erfindungsgemäß wird auch ein Verfahren zur Herstellung eines Behälters mit geringer Partikelemission bereit gestellt. Dieses Verfahren umfasst die Schritte:
a) Bereitstellen eines Behälterkörpers mit Außen- und einer Innenseite, wobei die Innenseite Siliziumoxid-haltig ist,
b) Aufbringen eines Gemisches aus einer fluororganischen Verbindung gelöst in einem Lösungsmittel auf zumindest einem Teil der Innenseite des wobei die fluorhaltige Verbindung eine Alkoxysilan-Verbindung laut Anspruch 5 ist,
c) Trocknung und Vernetzung der fluorhaltigen Verbindung mit der Siliziumoxid-haltigen Oberfläche an der Innenseite des Behälterkörpers mittels einer Kondensationsreaktion, sowie Quervernetzung der fluorhalitgen Verbindungen untereinander mittels der Verbinders.

Ein weiteres Merkmal des Verfahrens ist, dass die fluorhaltigen Verbindungen untereinander quer vernetzen.

Die Konzentration der in Schritt b) verwendeten fluororganischen Verbindung liegt im Bereich 0,01% bis 1%, bevorzugt im Bereich 0,03% bis 0,5%, besonders bevorzugt im Bereich 0,05% bis 0,3%.

In Schritt b) kann ein fluorhaltiges Lösungsmittel verwendet werden, das zumindest eine der folgenden Verbindungen enthält:
- Ethoxynonafluorbutan,
- Methoxynonafluorbutan,
- Perfluorhexan,
- Hydrofluorether,
- Solvay Solexis HAT-110,
- Fluorinert FC-77,
- Perfluorosolv PFS-1,
- Perfluorosolv PFS-2.

Die Vernetzung kann unter direkter Einwirkung von Temperatur und/oder Wasser, insbesondere einer feuchten Gasatmosphäre, oder unter Einwirkung einer wässrigen Lösung, insbesondere einer sauren Lösung erfolgen.

Die Vernetzung erfolgt bei einer Temperatur oberhalb von 30°C oder einer relativen Luftfeuchtigkeit im Bereich 10% bis 95%, besonders bevorzugt im Bereich 30% bis 70%. Hierzu wird das beschichtete Pharmapackmittel in einen Klimaschrank gebracht, in dem die Werte für relative Luftfeuchtigkeit und Temperatur vorgegeben werden können.

Alternativ kann die Vernetzung auch an Luft unter Einwirkung von Luftfeuchtigkeit aus der Umgebung erfolgen.

Im Schritt des Bereitstellens des Behälterkörpers kann die Innenseite der Behälteroberfläche zumindest teilweise vorbehandelt werden, wobei das Vorbehandeln in zumindest einem der folgenden Schritte durchgeführt wird:
- Thermisches Vorbehandeln bei einer Temperatur oberhalb 350 °C, bevorzugt oberhalb 400 °C, besonders bevorzugt oberhalb 500 °C,
- Waschen mit sterilem, partikelarmen Wasser,
- nass-chemisches Vorbehandeln mit einer sauren oder einer basischen Lösung,
- Reinigen gemäß einem der beiden vorstehenden Schritte unter Anwendung von Ultraschall mit einer Frequenz im Bereich 20 kHz bis 2,5 MHz, bevorzugt mit einer Frequenz im Bereich 100 kHz bis 2 MHz,
- Trocknen, vorzugsweise mittels Einblasen von Luft oder in einem Durchlaufofen.

Zwischen den Schritten des Bereitstellens des Behälterkörpers und des Aufbringens eines Gemisches kann in einem zusätzlich Schritt a1) eine Zwischenschicht auf zumindest einem Teilbereich der Innenseite des Behälterkörpers aufgebracht werden, wobei die Zwischenschicht mindestens eines der folgenden Merkmale aufweist:
- Die Zwischenschicht fungiert als Haftvermittler-Schicht,
- die Zwischenschicht enthält Siliziumoxid,
- die Zwischenschicht ist Sol Gel-basiert,
- die Zwischenschicht enthält wenigstens eine unter- oder überstöchiometrische Oxidverbindung,
- die Zwischenschicht ist mit weiteren Verbindungen dotiert,
- die Zwischenschicht umfasst ein Mischoxid, bevorzugt ein dotiertes Siliziumoxid, besonders bevorzugt ein mit einem Oxid der Elemente Al, Mg, P, Ce, Zr, Ti, Ba, Sr, Nb, B oder mit Magnesiumfluorid dotiertes Siliziumoxid.

Zur weiteren Verbesserung der Eigenschaften der Beschichtung kann die Innenseite der Behälteroberfläche ergänzend nach Schritt c) zumindest teilweise nachbehandelt werden, wobei das Nachbehandeln in zumindest einem der folgenden Schritte durchgeführt wird:
- Trocknen unter Umgebungsbedingungen oder in einem Ofen,
- Nachreinigen mittels Ultraschallbad und/oder unter Verwendung eines Lösungsmittels und/oder unter Verwendung eines fluorhaltigen Lösungsmittels und/oder unter Verwendung des gleichen Lösungsmittels wie in Schritt b) und/oder unter Verwendung von Wasser, bevorzugt von Water for Injection (WFI).

Das Aufbringen des Gemisches kann durch eine Flüssigbeschichtung erfolgen, wobei die Flüssigbeschichtung mittels eines der folgenden Verfahren erfolgt:
- Sprühverfahren,
- Tauchverfahren,
- Abzugsverfahren,
- Aufwischverfahren,
- Flutverfahren,
- Durchflussverfahren.

Beim Sprühverfahren erfolgt die Flüssigbeschichtung mittels einer Zweistoffdüse oder einer Einstoffdüse, bevorzugt einer "Diving Nozzle" (d.h. einer eintauchenden Düse, die während des Sprühvorgangs in den Behälter eintaucht) oder einem Ultraschallzerstäuber.

Der erfindungsgemäße Behälter wird bevorzugt als Spritzensystem mit reibkontrollierter Oberfläche zur Aufbewahrung von pharmazeutischen Wirkstofflösungen, insbesondere von Protein- und Antikörper- basierten pharmazeutischen Wirkstoffformulierungen verwendet.

Im Folgenden wird die Erfindung unter Bezugnahme auf die beigefügten Zeichnungen und beispielhafte Ausführungsformen detaillierter beschrieben.

In den Zeichnungen zeigen
- Fig. 1: Haft- und Gleittreibungswerte für eine erfindungsgemäß beschichtete Glasspritze,
- Fig. 2: Werte für den Kontaktwinkel mit Wasser für eine erfindungsgemäß beschichtete Glasspritze,
- Fig. 3: die Losbrechkraft an der Innenseite für eine erfindungsgemäß beschichtete Glasspritze in Abhängigkeit von der Lagerungsdauer und dem Lagermedium,
- Fig. 4: die Gleitreibungskraft an der Innenseite einer erfindungsgemäß beschichteten Glasspritze in Abhängigkeit von der Lagerungsdauer und dem Lagermedium,
- Fig. 5a: die Partikelkonzentration für eine erfindungsgemäß beschichtete Glasspritze im Vergleich zu einer silikonisierten und einer unbeschichteten Glasspritze,
- Fig. 5b: einen vergrößerten Ausschnitt von Fig. 5a,
- Fig. 6: den Kontaktwinkel mit Wasser einer erfindungsgemäß beschichteten Glasspritze mit und ohne thermische Vorbehandlung,
- Fig. 7: die Gleitreibung einer erfindungsgemäß beschichteten Glasspritze mit und ohne thermische Vorbehandlung,
- Fig. 8: eine Pareto-Analyse des statistischen Versuchsplans für die Zielgröße "Kontaktwinkel mit Wasser" nach Einlagerung mit Wasser für 28 Tage bei 40°C,
- Fig. 9: eine Pareto-Analyse des statistischen Versuchsplans für die Zielgröße "Kontaktwinkel mit Wasser" nach Einlagerung mit einem Phosphatpuffer für 28 Tage bei 40°C,
- Fig. 10: eine Pareto-Analyse des statistischen Versuchsplans für die Zielgröße "Gleitreibung" nach Einlagerung mit Wasser für 28 Tage bei 40°C,
- Fig. 11: eine Pareto-Analyse des statistischen Versuchsplans für die Zielgröße "Gleitreibung" nach Einlagerung mit einem Phosphatpuffer für 28 Tage bei 40°C,
- Fig. 12: die Gleitreibungskraft erfindungsgemäß beschichteter Glasspritzen in Abhängigkeit vom Beschichtungsverfahren,
- Fig. 13: die Losbrechkraft erfindungsgemäß beschichteter Glasspritzen in Abhängigkeit vom Beschichtungsverfahren, und
- Fig. 14: eine Kraft-Weg-Kurve von mittels DC2634 beschichteter Glasspritzen

Erfindungsgemäß wird ein Behälter bereit gestellt, der insbesondere ein Spritzen- oder Karpulensystem zur Aufbewahrung und/oder Verabreichung von medizinischen Wirkstoffen in flüssiger Form oder ein Pharmafläschchen (Vial) zur Aufbewahrung derartiger Wirkstoffe ist. Ein solcher Behälter umfasst einen Glaskörper mit einer Außen- und einer Innenseite, wobei letztere eine zumindest teilweise modifizierte Oberfläche aufweist. Der Glaskörper gehört zur hydrolytischen Klasse I oder II.

Alternativ kann der Behälter auch einen Kunststoffkörper umfassen, vorzugsweise auf Basis von Cycloolefinpolymer (COP) oder Cycloolefincopolymer (COC), der auf seiner Innenseite eine zumindest teilweise modifizierte glasartige Beschichtung aufweist.

Ferner umfasst der Behälter einen Elastomerstopfen, der in Reibpaarung mit der modifizierten Oberfläche der Innenseite steht. Auch kann der Behälter einen Verschluss, wie zum Beispiel ein TipCap aus Elastomer umfassen.

Das Glas oder die glasartige Oberfläche hat einen Anteil an Siliziumoxid von mehr als 50%, bevorzugt mehr als 60% und besonders bevorzugt mehr als 65%.

Die zumindest teilweise Modifikation der Oberfläche erfolgt mittels einer fluorhaltigen Verbindung, die zumindest teilweise chemisch über (Si-O-Si)-Bindungen an die Siliziumoxid-haltige Innenoberfläche gebunden ist. Bei der auf die Oberfläche aufgebrachten fluorhaltigen Verbindung handelt es sich um eine monopodale fluorhaltige Alkoxysilan-Verbindung. Die chemische Anbindung an das Glas erfolgt über eine Kondensationsreaktion.

Die chemische Anbindung der Alkoxysilan-Verbindung hat gegenüber einer nur aufgesprühten Substanz, wie zum Beispiel einem aufgesprühten Öl aus perfluorierten Polyether (PFPE), den Vorteil einer erhöhten Stabilität der Beschichtung und verbesserten Anbindung ans Substrat.

Die Alkoxysilan-Verbindung hat den folgenden prinzipiellen Aufbau:

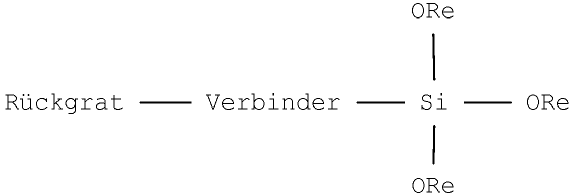

Die Abkürzung "ORe" steht für eine Alkoxygruppe.

Die Alkoxysilan-Verbindung weist mindestens eines der folgenden Merkmale auf.

Das Rückgrat oder Backbone der Alkoxysilan-Verbindung wird aufgrund der chemischen Anbindung des Alkoxysilans an die Oberfläche in der Richtung der Oberfläche ausgerichtet, was zur Folge hat, dass das Rückgrat beweglich bleibt, was die Reibeigenschaften der Oberfläche verbessert.

Das Rückgrat ist fluorhaltig. Insbesondere kann dieses Rückgrat Perfluorpolyether enthalten.

Zusätzlich umfasst das fluorhaltige Rückgrat mindestens eine (CF₂)₃-Kette und optional mehrere (CF₂)ₓ-Einheiten, für die alle x < 8 gilt.

Auch kann das fluorhaltige Rückgrat [(CF₂)ₓO]ₙ mit 3 < n < 1000, bevorzugt mit 4 < n < 200, besonders bevorzugt mit 5 < n < 100 enthalten.

Das Rückgrat kann auch weitere Verzweigungen enthalten. Ferner kann es CF₃-Gruppen als weitere Verzweigungen enthalten, und/oder lineare, verzweigte und/oder zyklische Strukturen.

Das Rückgrat kann abgesättigt oder unabgesättigt sein.

Die Alkoxysilan-Verbindung kann mindestens eine CF₃-Endgruppe enthalten.

Vorzugsweise besteht die Alkoxysilan-Verbindung aus einem Methoxysilan, so dass, beispielweise im Vergleich zu einem Ethoxysilan, die Verbindung eine höhere chemische Reaktivität aufweist.

Zwischen dem Rückgrat und der Alkoxy-Silan-Gruppe kann sich ein Verbinderbefinden. Aufgabe dieses Verbinders ist es, 2 oder mehrere benachbarte alkoxysilanhaltige Moleküle untereinander quer zu vernetzen, was die Stabilität der auf die Oberfläche aufgebrachten Schicht erhöht.

Der Verbinder enthält zumindest eine der folgenden Verbindungen:
- Eine hydrolisierbare Gruppe,
- eine Aminogruppe,
- eine Carbonsäureamidgruppe -OC-NH-,
- wenigstens eine weitere Siloxan-Gruppe,
- wenigstens eine weitere Silan-Gruppe,
- eine Acrylat- oder eine Methacrylat-Gruppe.

Ein wesentliches Merkmal des erfindungsgemäßen Behälters bzw. Pharmapackmittels ist die Reduzierung der Verunreinigung des Inhaltes des Packmittels durch Migration und durch minimierte Wechselwirkung von Bestandteilen der modifizierten Innenoberfläche in die medizinischen Wirkstoffe. Zur Vermeidung derartiger Verunreinigungen der medizinischen Wirkstoffe ist die modifizierte Innenoberfläche zumindest bis unmittelbar vor der Befüllung der Spritze oder des Fläschchens oder vor dem Setzen des Stopfens im Wesentlichen frei von fluiden oder mobilen Gleitölen und bildet somit eine Trockengleitoberfläche. Die modifizierte Innenoberfläche weist maximal 5 µg/cm², bevorzugt maximal 0,5 µg/cm², besonders bevorzugt 0,005 µg/cm² Masse Gleitöl, normiert auf 1 cm² modifizierter Innenoberfläche, auf.

Ferner ist die modifizierte Innenoberfläche im Wesentlichen frei von Polyorganosiloxan-Verbindungen, wie beispielweise Silikonölen. Dies bedeutet, dass die Oberfläche keine Polyorganosiloxan-Verbindungen, wie beispielsweise Polydimethylsiloxan-Verbindungen (PDMS) enthält. Letzteres wird definiert durch maximal 5 µg/cm², bevorzugt maximal 0,5 µg/cm², besonders bevorzugt 0,005 µg/cm² Masse derartiger Verbindungen, normiert auf 1 cm² modifizierter Innenoberfläche.

In einer ersten weiteren Ausführungsform ist der Behälterkörper auf seiner Innenoberfläche gänzlich frei von Polyorganosiloxan-Verbindungen. In einer zweiten weiteren Ausführungsform ist der Behälter auf seinen Innenoberflächen, die sich während der Lagerung in unmittelbarem Kontakt mit dem Produkt befinden, gänzlich frei von Polyorganosiloxan-Verbindungen.

In einer dritten weiteren Ausführungsform ist der Behälterkörper auf seiner Innen- und Außenoberfläche gänzlich frei von Polyorganosiloxan-Verbindungen.
In einer vierten weiteren Ausführungsform ist der Behälterkörper gänzlich frei von Polyorganosiloxan-Verbindungen. In einer fünften weiteren Ausführungsform ist der Behälter gänzlich frei von Polyorganosiloxan-Verbindungen.

Die Freiheit der modifizierten Oberfläche von Silikonölen verringert auch die Protein-Aggregation im Vergleich zu silikonisierten Spritzenkörpern. Ferner ist als weiterer Vorteil auch eine reduzierte Protein-Adsorption möglich.

Auch ist die auf die Innenoberfläche des erfindungsgemäßen Behälters bzw. Pharmapackmittels aufgebrachte Alkoxysilan-Verbindung immobilisiert, im Wesentlichen immobilisiert und insbesondere nicht migrierfähig. Dies wird dadurch definiert, dass maximal 1%, bevorzugt 0,1% und besonders bevorzugt 0,01% oder vorzugsweise 10 ppm der Alkoxysilan-Verbindung aus der Oberfläche in ein Lösungsmittel oder in eine wässrige Lösung migrieren können. Ferner lösen sich im Kontakt mit einer wässrigen Lösung, die ein Volumen V hat, an Metallionen oder Metalloxidionen oder an Metalloxidhaltigen Partikeln aus der modifizierten Oberfläche maximal 1000 ppb, bevorzugt maximal 500 ppb und besonders bevorzugt maximal 50 ppb von Metallionen, wie beispielsweise Wolframhaltigen Ionen.

Von Partikeln, die während der Glasherstellung entstanden sind, wie beispielweise Siliziumoxid- oder Borat- oder metallhaltigen Partikeln lösen sich maximal 10.000 Partikel pro ml aus der modifizierten Oberfläche, wobei die Partikel einen Durchmesser ≥ 2µm haben.

Insgesamt ergibt sich in Abhängigkeit vom Partikeldurchmesser die folgende Verteilung der Anzahl von in eine wässrige Lösung migrierten Partikeln, wobei die Messung mit einem Hyac-Roco Partikeltester durchgeführt wurde:
- weniger als 10.000 Partikel mit einem Durchmesser ≥ 2 µm,
- weniger als 3.000 Partikel mit einem Durchmesser ≥ 5 µm,
- weniger als 100 Partikel mit einem Durchmesser ≥ 10 µm,
- weniger als 10 Partikel mit einem Durchmesser ≥ 25 µm und
- weniger als 5 Partikel mit einem Durchmesser ≥ 50 µm.

Bevorzugt ergibt sich die folgende Verteilung der Anzahl von in eine wässrige Lösung migrierten Partikeln:
- weniger als 1.000 Partikel mit einem Durchmesser ≥ 2 µm,
- weniger als 300 Partikel mit einem Durchmesser ≥ 5 µm,
- weniger als 50 Partikel mit einem Durchmesser ≥ 10 µm,
- weniger als 8 Partikel mit einem Durchmesser ≥ 25 µm und
- weniger als 3 Partikel mit einem Durchmesser ≥ 50 µm.

Bezogen auf einen ml wässriger Lösung konnte die folgende Verteilung gemessen werden:
- weniger als 300 Partikel/ml mit einem Durchmesser ≥ 2 µm,
- weniger als 70 Partikel/ml mit einem Durchmesser ≥ 5 µm,
- weniger als 30 Partikel/ml mit einem Durchmesser ≥ 10 µm,
- weniger als 5 Partikel/ml mit einem Durchmesser ≥ 25 µm und
- weniger als 2 Partikel/ml mit einem Durchmesser ≥ 50 µm.

Die Reibungsmerkmale der modifizierten Oberfläche werden im Folgenden durch einen Vergleich der an einer nicht modifizierten Oberfläche auftretenden Haft- und Gleitreibungskräfte charakterisiert.

Im Vergleich zu einer nicht modifizierten Oberfläche ist die Haft- oder Gleitreibung um 1 N, bevorzugt um 5 N reduziert. Die Streuung der Haft- und Gleitreibung ist um mindestens 0,5 N, bevorzugt um mindestens 1 N vermindert.

Im unbefüllten, d.h. trockenen Zustand weist die modifizierte Oberfläche eine Haftreibung unterhalb von 20 N, bevorzugt unterhalb von 15 N und besonders bevorzugt unterhalb von 15 N auf. Die Gleitreibung liegt unterhalb von 10 N, bevorzugt unterhalb von 8 N und besonders bevorzugt unterhalb von 4 N. Die Streuung der Haft- und Gleitreibung liegt unterhalb von ± 4 N, bevorzugt unterhalb von ± 2 N und besonders bevorzugt unterhalb von ± 1 N. Die genannten Werte gelten auch während des Setzprozesses des Stopfens nach dem Abfüllen.

Im "nassen", d.h. mit Wasser befüllten Zustand weist die modifizierte Oberfläche eine Haftreibung unterhalb von 20 N, bevorzugt unterhalb von 15 N und besonders bevorzugt unterhalb von 10 N auf. Die Gleitreibung liegt unterhalb von 6 N, bevorzugt unterhalb von 4 N und besonders bevorzugt unterhalb von 3 N. Die Streuung der Haft- und Gleitreibung liegt unterhalb von ± 2 N, bevorzugt unterhalb von ± 1 N und besonders bevorzugt unterhalb von ± 0,5 N. Die genannten Werte gelten auch während der Injektion des medizinischen Wirkstoffs.

Die aufgeführten Werte für die auftretenden Reibungskräfte wurden vorzugsweise mit einem FluroTec-Stopfen Westar RU, B2-40 bei einer Hubgeschwindigkeit des Kolbenstopfens von 100 mm/min gemessen, vorzugsweise mit einem Instron-Messgerät. Die hierbei verwendeten Nadeln waren vorzugsweise eine 27G x 1/2" oder eine 29G x 1/2" Nadel.

Ein weitere potentielle Quelle von Verunreinigungen der medizinischen Wirkstoffe besteht darin, dass der Stopfen des Packmittels in Reibpaarung mit der modifizierten Innenoberfläche steht und daher bei einer Bewegung des Stopfens entlang der Oberfläche Partikel aus der Oberfläche abgelöst werden und in die medizinischen Wirkstoffe migrieren und diese dadurch verunreinigt werden.

Die modifizierte Oberfläche weist das Merkmal auf, dass nach einer oder mehreren Hubbewegungen des in Reibpaarung zur Oberfläche stehenden Elastomerstopfens von der modifizierten Oberfläche maximal 2.000 Partikel pro cm² Oberfläche abgelöst werden, wobei die Partikel einen Durchmesser ≥ 2 µm aufweisen. Gemessen wird dieses Merkmal bevorzugt im Rasterelektronenmikroskop.

Befindet sich zwischen der modifizierten Innenoberfläche und dem Elastomerstopfen ein weiterer Reibpartner, beispielsweise eine wässrige Lösung oder eine Pufferlösung, so werden von der modifizierten Oberfläche maximal 2.000 Partikel pro cm² Oberfläche abgelöst, wobei die Partikel einen Durchmesser ≥ 2 µm aufweisen. Gemessen wird dieses Merkmal bevorzugt im Rasterelektronenmikroskop.

Die Schichtdicke der fluororganischen Schicht liegt im Bereich 0,1 nm bis 40 nm, bevorzugt im Bereich 0,5 nm bis 10 nm und besonders bevorzugt für eine Monolage im Bereich bis maximal 10 nm.

Ein weiterer Parameter für die Wechselwirkung der erfindungsgemäß modifizierten Oberfläche mit Wasser ist der Kontaktwinkel für Wasser. Dieser liegt für die erfindungsgemäße Beschichtung oberhalb von 100°, bevorzugt oberhalb von 105° und besonders bevorzugt oberhalb von 110°.

Der dynamische Kontaktwinkel der Schichtoberfläche beträgt beim Eintauchen (Fortschrittswinkel) ≥ 110°, bevorzugt ≥ 115° und im Rückzug (Rückzugswinkel) ≥ 115°, bevorzugt ≥ 105°.

Für ein Tröpfchen mit einem Volumen von 60 µl liegt der Abrollwinkel der Schicht im Bereich von 1° bis 30°, bevorzugt im Bereich von 5° bis 20°.

Der Glaskörper der Spritze weist eine Kanüle auf. Wird die Innenoberfläche der Kanüle nicht beschichtet, so erhöht dies die Anhaftung einer in die Kanüle eingeklebten oder angebundenen Nadel. Gemessen wird dies durch einen Nadelauszugstest, bei dem Nadelauszugskraft oberhalb von 10 N, bevorzugt oberhalb von 22 N liegt.

Da die Außenoberfläche des Behälters bzw. Pharmapackmittels nicht beschichtet wird, erhöht dies die Anhaftkraft eines auf der Außenoberfläche angebrachten Klebeetiketts.

Erfindungsgemäß wird auch ein Verfahren zur Herstellung eines Behälters mit geringer Partikelemission bereit gestellt.

In einem ersten Schritt wird ein Behälterkörper mit einer Außen- und einer Innenseite bereit gestellt. Die Innenseite enthält Siliziumoxid.

Die erfindungsgemäße Beschichtung erfolgt durch das Aufbringen eines Gemisches aus einer fluororganischen Verbindung gelöst in einem Lösungsmittel auf zumindest einem Teil der Innenseite des Behälterkörpers.
Das hierbei verwendete Lösungsmittel ist fluorhaltig und nicht ozon-schädlich ("zero ozone depletion potential").

Das Lösungsmittel beziehungsweise die verdünnte Lösung aus Lösungsmittel und fluororganischer Verbindung haben einen Siedepunkt im Bereich von 30°C bis 200°C, bevorzugt im Bereich von 40°C bis 95°C und besonders bevorzugt im bereich von 50°C bis 80°C.

Die Konzentration der fluororganischen Verbindung in dem Lösungsmittel liegt im Bereich von 0,01% bis 1%, bevorzugt im Bereich von 0,03% bis 0,5% und besonders bevorzugt im Bereich 0,05% bis 0,3%.

Das für das erfindungsgemäße Verfahren verwendete Lösungsmittel enthält mindestens eine der folgenden Verbindungen:
- Ethoxynonafluorbutan (3M Novec HFE7200, C₄F₉OC₂H₅),
- Methoxynonafluorbutan (HFE-7100, C₄F₉OCH₃ bestehend aus 2 den Isomeren (CF₃)₂CFCF₂OCH₃ und CF₃CF₂CF₂CF₂OCH₃,
- Perfluorohexan,
- Hydrofluoroether,
- Solvay Solexis HAT-110,
- Fluorinert FC-77,
- Perfluorosolv PFS-1 oder
- Perfluorosolv PFS-2.

In einem weiteren Schritt erfolgt die Trocknung und Vernetzung der fluorhaltigen Verbindung mit der Siliziumoxid-haltigen Oberfläche an der Innenseite des Behälterkörpers mittels einer Kondensationsreaktion.

Die Vernetzung der Schicht erfolgt an einer feuchten Gasatmosphäre oder unter direkter Einwirkung von Wasser, einer wässrigen Lösung oder speziell auch unter Einwirkung einer sauren Lösung.

Die relative Luftfeuchtigkeit während der Vernetzung der Schicht liegt im Bereich von 10% bis 95%, bevorzugt im Bereich von 30% bis 70%.

Die Vernetzung der Schicht kann auch unter Einwirkung von Luftfeuchtigkeit aus der Umgebung erfolgen.

Während der Trocknung verdampfen mindestens zwei volatile Verbindungen aus der Schicht im Bereich zwischen Raumtemperatur und 250°C.

Ferner kann die Vernetzung der Schicht während eines simultanen Sterilisationsprozesses, beispielweise einer ETO-Sterilisation erfolgen.

In einer Ausführungsform enthält die auf die Innenoberfläche des Behälters bzw. Pharmapackmittels aufgebrachte Lösung weitere Additive oder Vernetzer, beispielsweise Vernetzer, die mit UV-Licht oder thermisch aktivierbar sind.

In einer weiteren Ausführungsform des Verfahrens kann das Glas oder die glasartige Oberfläche des Behälters bzw. Pharmapackmittels vor der eigentlichen Beschichtung vorbehandelt werden. Hierdurch können gebundenes Wasser oder organische Verbindungen aus der Glasoberfläche entfernt werden.

Für die Vorbehandlung wird mindestens eines der im Folgenden beschriebenen Verfahren eingesetzt werden.

Das Glas wird bei einer Temperatur oberhalb von 350°C, bevorzugt oberhalb von 400°C und besonders bevorzugt oberhalb von 500°C thermisch vorbehandelt.

Die Oberfläche kann mit sterilem, partikelarmem Wasser gewaschen werden.

Die Oberfläche kann auch mit einer sauren oder einer alkalischen Lösung nass-chemisch vorbehandelt werden.

Die beiden letztgenannten Vorbehandlungsverfahren können zusätzlich in einem Ultraschallbad durchgeführt werden. Der eingesetzte Ultraschall hat eine Frequenz im Bereich von 20 kHz bis 2,5 MHz, bevorzugt im Bereich 100 kHz bis 2 MHz.

Nach einer Vorbehandlung mittels eines der beschriebenen Verfahren erfolgt eine Trocknung der Oberfläche, vorzugsweise mittels eingeblasener Luft oder in einem Durchlaufofen.

Es konnte nachgewiesen werden, dass eine derartige Vorbehandlung die Lagerstabilität der Beschichtung signifikant erhöht, was im Zusammenhang mit der späteren Beschreibung von Ausführungsbeispielen näher erläutert wird.

Die Vorbehandlung des Glases beziehungsweise der glasartigen Oberfläche führt zu einer Entfernung der Wasserhaut und der Organik auf der Oberfläche, wobei vor der Beschichtung der Kontaktwinkel für Wasser kleiner als 50°, vorzugsweise kleiner als 20° ist.

In einem weiteren Schritt des erfindungsgemäßen Verfahrens wird zur Erhöhung der Schichtstabilität nach dem Bereitstellen des Behälters eine Zwischenschicht auf der Oberfläche aufgebracht.

Diese Zwischenschicht kann als Haftvermittlerschicht fungieren. Sie kann Siliziumoxid enthalten. Auch kann die Zwischenschicht Sol Gel-basiert sein. Zusätzlich oder alternativ kann die Zwischenschicht wenigstens eine unter- oder überstöchiometrische Oxidverbindung enthalten. Ferner kann diese Zwischenschicht mit weiteren Verbindungen dotiert sein.

In einer bevorzugten Ausführungsform umfasst die Zwischenschicht ein Mischoxid, bevorzugt ein dotiertes Siliziumoxid. In einer bevorzugten Ausführungsform ist das Siliziumoxid mit einem Oxid der folgenden Elemente dotiert: Aluminium, Magnesium, Phosphor, Cer, Zirkon, Titan, Barium, Strontium, Niob, Bor. Das Siliziumoxid kann auch mit Magnesiumfluorid dotiert sein.

In einer weiteren Ausführungsform des erfindungsgemäßen Verfahrens wird die Beschichtung in einem abschließenden Schritt nachbehandelt, indem die Schicht nach einer Trocknung unter Umgebungsbedingungen oder in einem Ofen nachgereinigt wird.

Diese Nachreinigung kann in einem Ultraschallbad erfolgen. Es kann hierbei ein Lösungsmittel verwendet werden, das fluorhaltig sein kann. Das Lösungsmittel kann das gleiche wie das für die Beschichtung verwendete sein. Auch Wasser, beispielsweise Water for Injection (WFI) kann für die Nachreinigung eingesetzt werden.

Das Aufbringen der Schicht selbst erfolgt mittels einer Flüssigbeschichtung. Als Verfahren zur Flüssigbeschichtung können ein Sprüh-, ein Tauch-, ein Abzugs-, ein Aufwisch-, ein Flut- oder ein Durchflussverfahren eingesetzt werden. In einer bevorzugten Ausführungsform erfolgt die Sprühbeschichtung mittels einer Zweistoffdüse oder einer Einstoffdüse, wie beispielsweise einem Ultraschallzerstäuber.

Eine homogene und lokale Beschichtung im Innenbereich einer Spritze wird insbesondere durch die Verwendung einer Diving Nozzle erreicht.

Das für die Beschichtung mittels Sprühprozess verwendete Sprühvolumen liegt im Bereich von 0,1 µl bis 500 µl, bevorzugt im Bereich von 3 µl bis 150 µl und besonders bevorzugt im Bereich von 20 µl bis 100 µl.

Beim Einsatz einer Zweistoffdüse wird ein Sprühdruck im Bereich von 0,1 bar bis 5 bar, bevorzugt im Bereich von 0,2 bar bis 2,5 bar und besonders bevorzugt im Bereich von 0,5 bar bis 1,5 bar eingesetzt. Der Gasfluss beträgt bei Verwendung einer Zweistoffdüse 0,1 bis 50 l/min, bevorzugt 0,5 bis 20 l/min und besonders bevorzugt 2 bis 5 l/min.

Die Sprührate für den Sprühprozess zum Aufbringen der Beschichtung beträgt 0,01 µl/s bis 100 µl/s, bevorzugt 25 µl/s bis 100 µl/s. Bei der letztgenannten Sprührate ergibt sich eine Sprühzeit von 0,5 s bis 4 s.

Aufgrund der sehr kurzen Sprühzeit wird eine hohe Produktivität beim erfindungsgemäßen Herstellprozess erreicht.

Wird eine Diving Nozzle eingesetzt, so liegt deren Öffnungsdurchmesser im Bereich von 0,1 mm bis 1 mm. Bezogen auf die Höhe des Spritzen- oder Karpulenzylinders, beziehungsweise auf die Höhe des Vial Zylinders oder der Ampulle liegt die Eintauchtiefe der Düse im Bereich 10% bis 95%, bevorzugt im Bereich 30% bis 90% und besonders bevorzugt im Bereich 45% bis 85%.

Hierbei kann die Sprühdüse entweder vertikal von oben oder von unten oder horizontal in das innenseitig zu beschichtende Pharmapackmittel eingefahren werden.

Zum Aufsprühen wird eine Lösung mit einer kinematischen Viskosität, gemessen bei Raumtemperatur und Normaldruck, im Bereich von 0,01 bis 10.000 Centi Stokes, bevorzugt im Bereich von 0,03 bis 100 Centi Stokes, besonders bevorzugt im Bereich von 0,05 bis 20 Centi Stokes und ganz besonders bevorzugt im Bereich von 0,1 bis 2 Centi Stokes verwendet.

In einer weiteren Ausführungsform des erfindungsgemäßen Verfahrens wird eine "staked-needle Spritze" (d.h. eine Spritze mit einer eingeklebten Nadel) beschichtet, indem die Beschichtung erst aufgebracht wird, nachdem die Nadel bereits in der Kanüle eingebracht worden ist. Es konnte festgestellt werden, dass bei dieser Vorgehensweise durch das Aufsprühen der Lösung die Verklebung der Nadel und insbesondere die Auszugskraft der Nadel nicht negativ beeinflusst.

Ein erfindungsgemäß beschichtetes Spritzensystem wird zur Aufbewahrung pharmazeutischer Wirkstofflösungen verwendet, insbesondere von protein-basierten pharmazeutischen Wirkstoffformulierungen. Aufgrund der erfindungsgemäßen Beschichtung wird Protein-Adsorption, -Aggregation oder Protein-Denaturierung im Vergleich zu einer herkömmlichen silikon-haltigen Spritze reduziert. Dies gilt insbesondere für den Fall, dass der pharmazeutische Wirkstoff Biomoleküle enthält, die gegenüber Silikonöl intolerant oder instabil sind.

Erfindungsgemäß kann der vorstehend beschriebene Behälter verwendet werden, um eine Medikamentenlösung aufzubewahren. Insbesondere die Verwendung des Behälters mit modifizierter Glasoberfläche für die Aufbewahrung von Lösungen mit Protein-basierten Wirkstoffen und/oder mit oberflächenaktiven Tensiden, die auch als 'Surfactants', wie beispielsweise Polysorbat, wie beispielsweise Tween20 oder Tween80 oder Pluronics, und/oder gepufferte oder ungepufferte Medikamentenlösungen, und/oder Formulierungen mit saurem oder neutralem oder alkalischem pH-Wert und/oder Formulierungslösungen die Zucker oder Zuckeralkohol enthalten. Ferner umfasst die Erfindung auch die Verwendung für die Aufbewahrung von Medikamentenlösungen beispielsweise in vorbefüllten Spritzen oder Karpulen, beispielsweise in Autoinjektoren oder medizinischen Vorrichtungen, die beispielsweise Medikamentenlösungen mit folgenden Bestandteilen:
Wässrige oder alkoholische Formulierungen, Biomoleküle, wie Peptide, Protein-Fragmente, Proteine, wie speziell monoklonale Antikörper, polyklonale Antikörper, Liganden, Rezeptoren, Antigene, Enzyme, die natürlich oder rekombinant hergestellt wurden als auch Derivative von diesen Biomolekülen. Spezifische Wirkstoff-Proteine umfassen dabei Antikörper (beispielsweise Remicade und ReoPro von Centocor; Herceptin von Genentech; Mylotarg von Wyeth, Synagis von MedImmune), Enzyme (beispielsweise Pulmozyme von Genentech; Cerezyme von Genzyme), rekombinante Hormone (beispielsweise Protropin von Genentech, Novolin von Zymogenetics, Humulin von Lilly), rekombinante Interferone (beispielsweise Actimmune von InterMune Pharmaceutical; Avonex von BiogenIdec, Betaseron von Chiron; Infergen von Amgen; Intron A von Schering-Plough; Roferon von Hoffman-La Roche), rekombinante Blutfaktoren (beispielsweise TNKase von Genentech; Retavase von Centocor; Refacto vom Genetics Institute; Kogenate von Bayer) and rekombinantes Erythropoietin (beispielsweise Epogen von Amgen; Procrit von J&J), ferner auch rekombinant hergestellte Fusionsproteine (beispielsweise Orencia / Abatacept von BMS) und Impfstoffe (beispielsweise Engerix-B von GSK; Recombivax HB von Merck & Co.). Ferner kann diese Schicht auch für andere biomolekulare Anwendungen verwendet werden, wie Nukleinsäuren, Polynukleotide wie DNA, RNA, pDNA, Oligonukleotide), Protein / Nukleinsäure Komplexe oder auch für Eisen-Saccharose-haltige Formulierungsbestandteile wie Eisen-Saccharose-Komplexe. Ferner auch Proteine mit einer aminoterminaler γ-Carboxyglutaminsäure(Gla)-Domäne mit 9-12 Gla-Resten, wie einem Vitamin-K abhängigen Blutgerinnungszymogenprotein oder ein aktivierte Form davon aus der Gruppe aus Prothrombin, Faktor VII, Faktor IX, Faktor X und Protein C, beispielsweise einem rekombinanten Human-Faktor VII (Novo Nordisk).

Im Folgenden werden einige Ausführungsformen des erfindungsgemäßen Behälters bzw. Pharmapackmittels beschrieben.

In einem ersten Ausführungsbeispiel ist das erfindungsgemäße Pharmapackmittel eine Glasspritze aus Borosilikatglas. Ein derartiges Glas wird beispielsweise unter dem Namen FIOLAX als Pharmarohrglas von der Anmelderin kommerziell angeboten. Die Spritze hat das Format 1 ml long und weist eine eingeklebte Nadel vom Typ 27Gx1/2'' auf.

Die Glasspritze wird zunächst mit Water for Injection (WFI) gereinigt und anschließend durch das Lösungsmittel HFE7200 in einem Ultraschallbad für 10 Minuten gereinigt. Für die nachfolgende Beschichtung der Innenseite der Spritze wird eine der kommerziell erhältlichen Beschichtungen Daikin AES4-E, Daikin DSX ("Optool") oder Dow Corning 2634 verwendet. Jede dieser drei Beschichtungen ist ein Perfluorpolyethersilan. Mittels des Lösungsmittels HFE7200 wird das zum Einsatz kommende Perfluorpolyethersilan auf eine Konzentration von 0,1% verdünnt und mit einem Magnetrührer verrührt.

Innenseitig wird die Glasspritze mit der verdünnten Lösung geflutet. Nach einer Einwirkdauer von 3 Minuten wird die überschüssige Beschichtungslösung wieder aus der Spritze entfernt. Anschließend wird die Spritze an Luft getrocknet, so dass das Lösungsmittel verdampft. In einem nächsten Schritt werden die Beschichtungen auf den Innenseiten der Spritzen in einem Klimaschrank bei einer Temperatur von 50 °C und einer relativen Luftfeuchtigkeit von 50% für eine Stunde vernetzt. Abschließend erfolgt eine Nachreinigung der Spritzen mit der nachvernetzten Beschichtung mittels HFE7200 in einem Ultraschallbad.

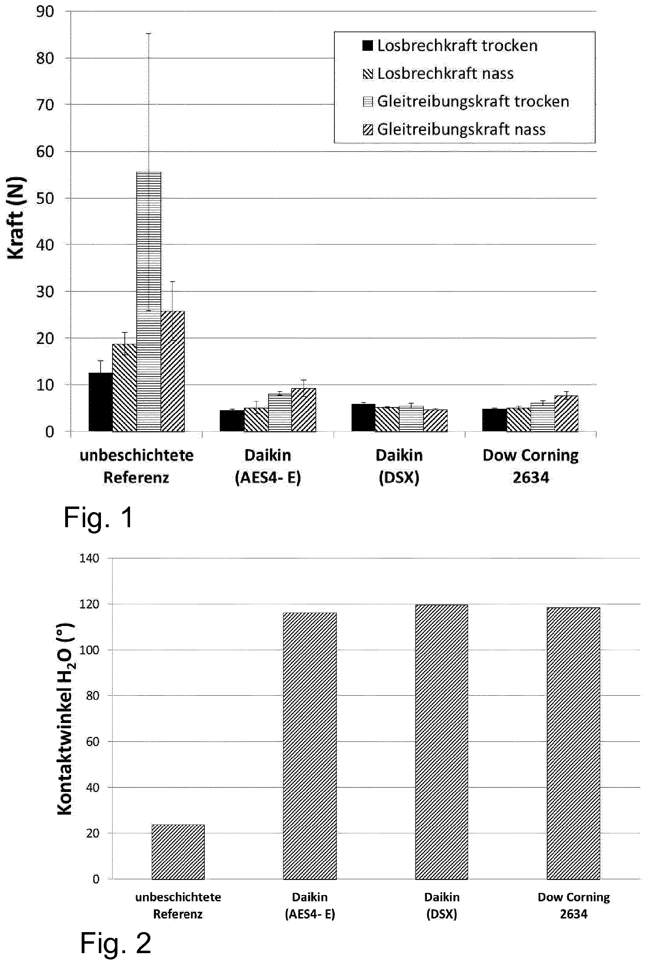

Fig. 1 zeigt unter Verwendung eines Flurotec Westar RU B2-40-Stopfens durchgeführte Messungen der Losbrechkraft- und Gleitreibungskraftwerte sowohl für unbefüllte ("trocken") und für mit Wasser befüllte ("nass") Spritzen bei einer Geschwindigkeit von 100 mm/min. Eine Glasspritze mit nicht beschichteter Innenseite dient als Referenzwert. Die Messungen wurden für jede der angeführten Beschichtungen durchgeführt. Die Messungen lassen eine wesentliche Reduzierung der Haft- und Gleitreibungswerte erkennen. Auch die Streuung dieser Werte ist deutlich reduziert. Beispielsweise wurde die Gleitreibung im Falle unbefüllter Spritzen auf weniger als ein Fünftel des Referenzwertes gesenkt.

Fig. 2 zeigt Messungen des Kontaktwinkels von Wasser. Im Falle von Glasspritzen mit beschichteter Innenseite liegt der Kontaktwinkel mit Wasser für alle drei Beschichtungen im Bereich von 115° bis 120°, während er für die unbeschichtete Referenzprobe lediglich bei etwa 25° liegt.

Für die Beschichtungen Daikin DSX ("Optool") und Dow Corning 2634 wurde untersucht, inwieweit sich die Reibungswerte in Abhängigkeit von der Dauer einer Lagerung der Spritzen sowie vom Lagermedium ändern. Hierzu wurde von den beschichteten Spritzen und unbeschichteten Referenzspritzen ein erster Teil mit Wasser und ein zweiter Teil mit Phosphatpuffer pH7 befüllt und bei einer Temperatur von 40 °C in einem beschleunigten Test eingelagert.

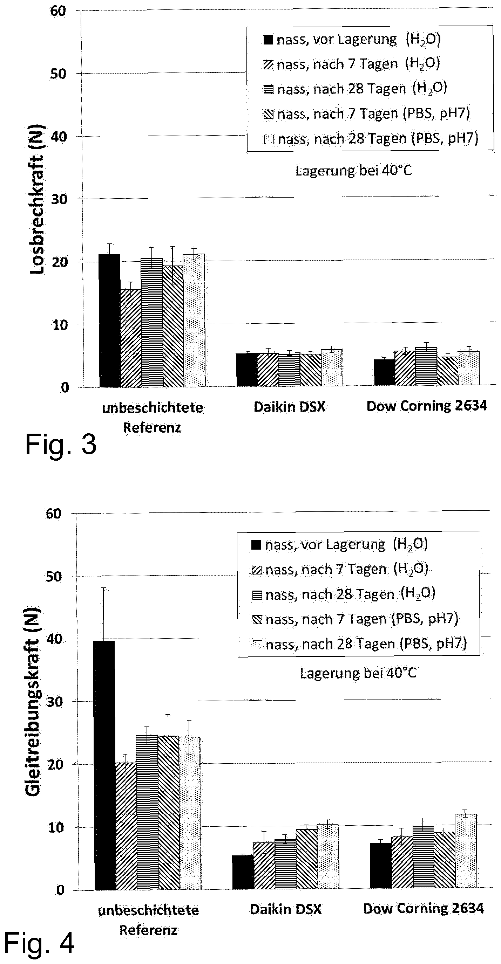

Fig. 3 zeigt die Losbrechkraft der Proben in Abhängigkeit von der Lagerungsdauer bei einer Temperatur von 40 °C und dem Lagermedium. Für jedes der beiden Lagermedien, Wasser und eine Phosphatpufferlösung mit pH7 (PBS pH 7) wurde die Losbrechkraft vor Beginn der Lagerung, nach einer Lagerdauer von 7 Tagen und nach einer Lagerdauer von 28 Tagen gemessen. Wie anhand von Fig. 3 ersichtlich, wird mit der erfindungsgemäßen Beschichtung eine dauerhafte Absenkung der Losbrechkraft der befüllten Spritzen erzielt.

Fig. 4 zeigt die Gleitreibung der Proben in Abhängigkeit von der Lagerungsdauer und dem Lagermedium. Die Gleitreibung wurde vor Lagerung mit Wasser. Für jedes der beiden Lagermedien (Wasser und Phosphatpuffer) wurde die Gleitreibung in einem beschleunigten Test bei einer Temperatur von 40°C nach einer Lagerdauer von 7 Tagen und nach einer Lagerdauer von 28 Tagen gemessen.

Die Messungen zeigen eine signifikante Verbesserung der Lagerstabilität der innenseitig beschichteten Probenspritzen sowohl für Wasser als auch für einen Phosphatpuffer im Vergleich zu einer unbeschichteten Probe. Fig. 3 und Fig. 4 zeigen, dass die geringen Haft- und Gleitreibungswerte der beschichteten Probenspritzen auch über eine längere Lagerungsdauer weitestgehend stabil bleiben.

In weiteren Messreihen wurden die erfindungsgemäß mit dem Perfluorpolyethersilan (Daikin DSX) beschichteten Glasspritzen mit Glasspritzen verglichen, die nach einem konventionellen Herstellungsprozess mit WFI mittels Sprühverfahren silikonisiert wurden.

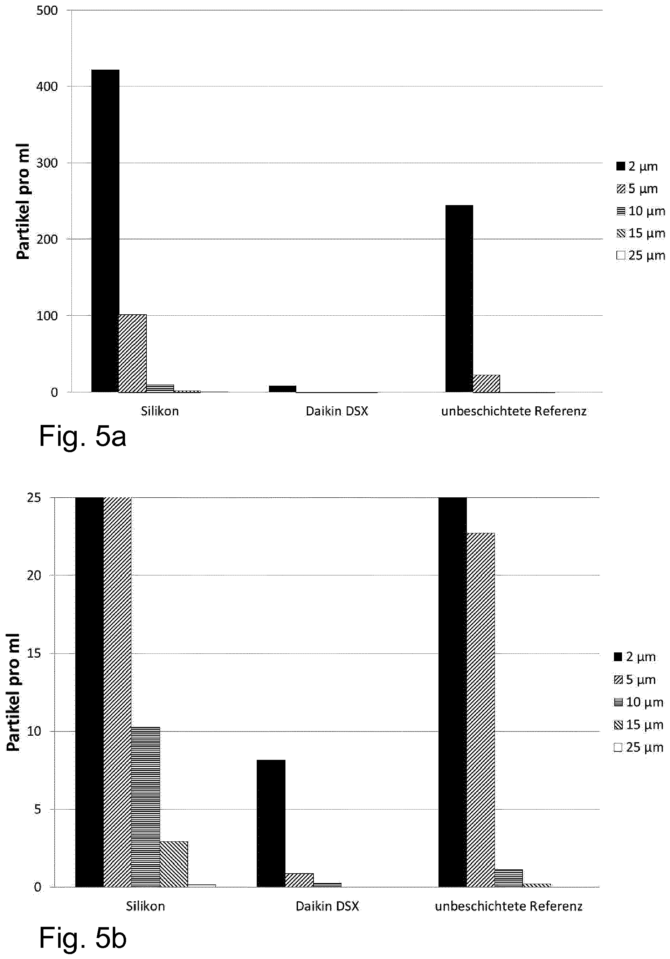

Fig. 5a zeigt die Anzahl der Partikel in wässriger Lösung für silikonisierte und erfindungsgemäß mit Perfluorpolyethersilan beschichtete Glasspritzen. Als Referenz dienen unbeschichtete Spritzen. Fig. 5a zeigt dabei die gemessene Gesamtpartikelgrößenverteilung der drei Spritzen. In Fig. 5b ist ein vergrößerter Ausschnitt von Fig. 5a gezeigt, um auch die äußert geringen Messwerte der erfindungsgemäß beschichteten Spritze ausmachen zu können.

Die dargestellten Messergebnisse zeigen, dass die Beschichtung der Glasspritzen mit Perfluorpolyethersilan eine wesentliche Abnahme der in wässriger Lösung emittierten nicht-sichtbaren Partikel bewirkt, verglichen mit einer silikonisierten Spritze sowie mit einer unbeschichteten Spritze. Insbesondere wird die Partikelemission für kleine nicht sichtbare Partikel mit Durchmessern ≤ 2 µm und ≤ 5 µm aufgrund der Beschichtung um mehr als einen Faktor 50 reduziert.

Die erfindungsgemäß beschichteten Glasspritzen stellen somit gegenüber herkömmlichen silikonisierten Spritzen hinsichtlich der Partikelemission eine wesentliche Verbesserung dar.

In einem zweiten Ausführungsbeispiel ist das erfindungsgemäße Pharmapackmittel ebenfalls eine Glasspritze aus Borosilikatglas. Die Spritze hat das Format 1 ml long und weist eine eingeklebte Nadel vom Typ 27Gx1/2'' auf.

Die Spritzen werden in einem folgenden Schritt thermisch bei 550°C für 30 Minuten, vorzugsweise an Luft vorbehandelt.

Für die nachfolgende Beschichtung der Innenseite der Spritze wird das kommerziell erhältliche Perfluorpolyethersilan Daikin DSX ("Optool") verwendet.

Mittels des Lösungsmittels HFE7200 wird das zum Einsatz kommende Perfluorpolyethersilan auf eine Konzentration von 0,1% verdünnt und mit einem Magnetrührer verrührt.

Innenseitig wird die Glasspritze mit der verdünnten Lösung geflutet. Nach einer Einwirkdauer von 3 Minuten wird die überschüssige Beschichtungslösung wieder aus der Spritze entfernt. Anschließend wird die Spritze an Luft getrocknet, so dass das Lösungsmittel verdampft.

In einem nächsten Schritt werden die Beschichtungen auf den Innenseiten der Spritzen in einem Klimaschrank bei Temperaturen von 20°C bis 70°C, einer relativen Luftfeuchtigkeit von 30% bis 90% für 0,5 bis 72 Stunden als Nachbehandlung vernetzt.

Anschließend werden die Spritzen mit der nachbehandelten Beschichtung mittels HFE7200 im Ultraschallbad nachgereinigt.

Dabei wird mittels eines statistischen Versuchsplans ein Screening-Versuchsplan unter Verwendung der Parameter thermische Vorbehandlung sowie der drei Nachbehandlungsparameter Temperatur, relative Luftfeuchtigkeit sowie Nachbehandlungsdauer durchgeführt.

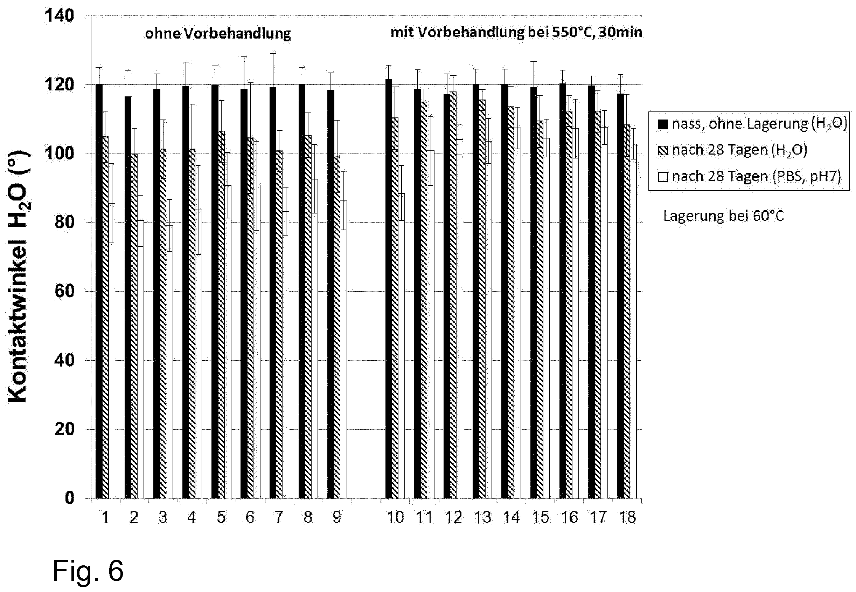

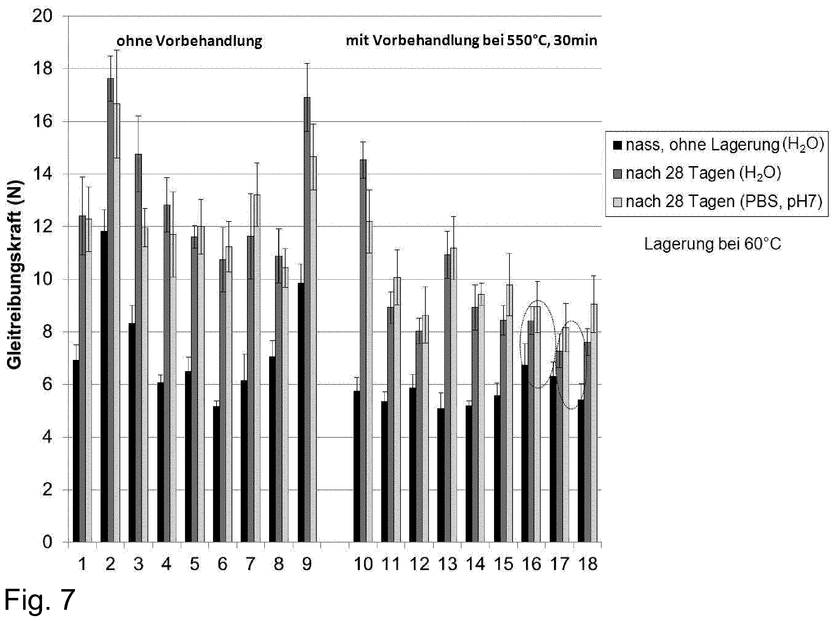

Darstellung der Parametervarianten 1-9 bzw. 10-18 zu Fig. 6 und Fig. 7:

| Probennummer | T(°C) | rel. Luftfeuchtigkeit (%) | t (h) |
|---|---|---|---|
| 1 | 45 | 60 | 36,3 |
| 2 | 20 | 90 | 0,5 |
| 3 | 20 | 30 | 72 |
| 4 | 70 | 90 | 0,5 |
| 5 | 70 | 30 | 72 |
| 6 | 20 | 90 | 72 |
| 7 | 70 | 30 | 0,5 |
| 8 | 70 | 90 | 72 |
| 9 | 20 | 30 | 0,5 |
| | | | |
| 10 | 20 | 30 | 0,5 |
| 11 | 70 | 30 | 0,5 |
| 12 | 70 | 30 | 72 |
| 13 | 20 | 90 | 0,5 |
| 14 | 20 | 90 | 72 |
| 15 | 70 | 90 | 0,5 |
| 16 | 20 | 30 | 72 |
| 17 | 70 | 90 | 72 |
| 18 | 45 | 60 | 36,3 |

Von den thermisch vorbehandelten und den thermisch nicht vorbehandelten Spritzen wird jeweils ein erster Teil der Proben mit Wasser und ein zweiter Teil mit einer Phosphatpufferlösung mit einem pH-Wert von 7 ('PBS pH 7') befüllt und in einem beschleunigten Test bei einer Temperatur von 60°C eingelagert.

Fig. 6 zeigt die Ergebnisse der Messung des Kontaktwinkels dieser Proben mit Wasser. Die Lagerdauer entsprach jeweils 28 Tagen.

Ferner wurden die Gleitreibungswerte der vorbehandelten und der nicht vorbehandelten Glasspritzen gemessen und verglichen. Die Ergebnisse dieser Messungen sind in Fig. 7 gezeigt. Die Gleitreibungswerte wurden unter Verwendung eines FluroTec Westar RU B2-40-Stopfens durchgeführt. Die Spritzen waren mit Wasser und Phosphatpufferlösung mit pH 7 befüllt und wie bei dem Beispiel gemäß Fig. 6 gelagert. Die Messungen erfolgten bei einer Geschwindigkeit von 100mm/min.

Wie die Messwerte in Fig. 7 zeigen, bewirkt eine thermische Vorbehandlung der Glasspritzen vor der Beschichtung mit einem Perfluorpolyethersilan eine signifikante Erhöhung des Kontaktwinkels mit Wasser, auch nach Auslagerung in Wasser oder einem Phosphatpuffer. Somit besitzen die Beschichtungen auf thermisch vorbehandelten Glasspritzen eine wesentlich höhere Lagerstabilität im Vergleich zu gleichartigen Beschichtungen auf thermisch nicht vorbehandelten Glasspritzen.

Ferner bewirkt eine thermische Vorbehandlung der Glasspritzen vor der Beschichtung mit einem Perfluorpolyethersilan eine deutliche Reduktion der Gleitreibungswerte, auch nach Auslagerung in Wasser oder einem Phosphatpuffer im Vergleich zu thermisch nicht vorbehandelten Glasspritzen. Hierdurch wird die höhere Stabilität der Schichten auf thermisch vorbehandelten Glasspritzen belegt. Aus den in Fig. 7 gezeigten Messwerten ergibt sich, dass bei den thermisch vorbehandelten Glasspritzen bei den Parametervarianten 16 und 17 ein Optimum vorliegt, da in diesen beiden Fällen die Gleitreibung nach Einlagerung nur geringfügig ansteigt.

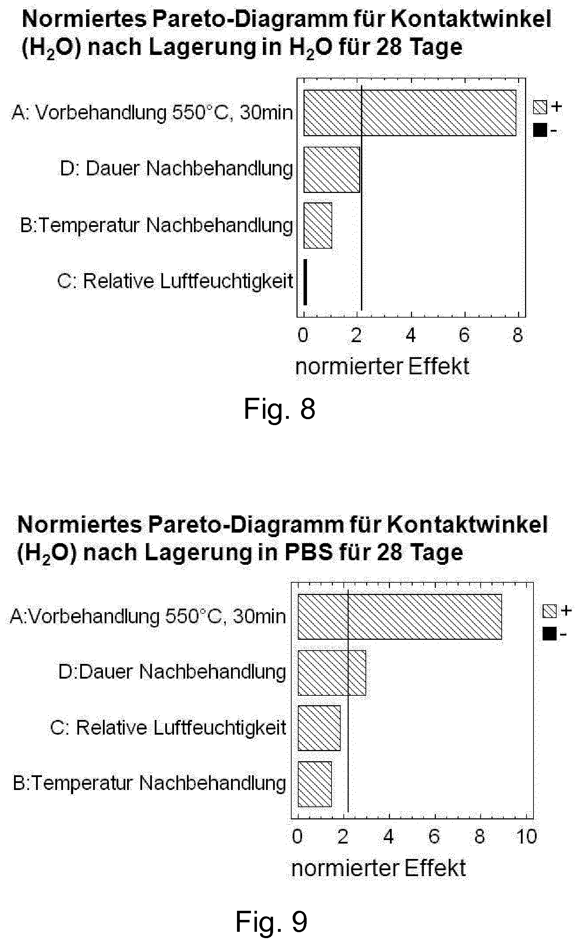

Diese Aussagen zur wesentlichen Verbesserung der Stabilität der Beschichtung werden durch die in den Fig. 8 und 9 gezeigten Pareto-Analysen des statistischen Versuchsplans belegt.

Fig. 8 zeigt die Ergebnisse einer Pareto-Analyse des statistischen Versuchsplans für die Zielgröße "Kontaktwinkel mit Wasser" nach einer Einlagerungszeit von 28 Tagen bei Befüllung der Glasspritzen mit Wasser bei 60°C.

Fig. 9 zeigt die Ergebnisse einer Pareto-Analyse des statistischen Versuchsplans für die Zielgröße "Kontaktwinkel mit Wasser" nach einer Einlagerungszeit von 28 Tagen bei Befüllung der Glasspritzen mit einem Phosphatpuffer mit einem pH-Wert von 7 bei 60°C.

Die Fig. 8 und 9 zeigen, dass die thermische Vorbehandlung der Glasspritzen vor der Beschichtung den Kontaktwinkel mit Wasser, gemessen nach einer Einlagerungszeit von 28 Tagen mit Wasser bzw. mit einem Phosphatpuffer bei 60°C statistisch signifikant erhöht, verglichen mit Glasspritzen, die thermisch nicht vorbehandelt wurden. Die Fig. 8 und 9 zeigen weiterhin, dass der Parameter "thermische Vorbehandlung" gegenüber den Nachbehandlungsparametern Temperatur, relative Luftfeuchtigkeit sowie Nachbehandlungsdauer die stärkere Einflussgröße ist.

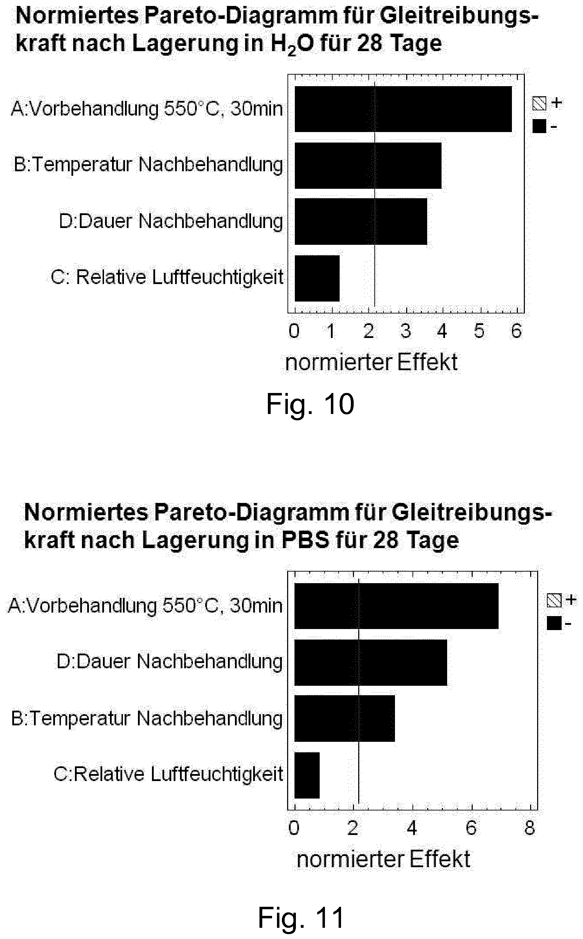

Die Fig. 10 zeigt die Ergebnisse einer Pareto-Analyse des statistischen Versuchsplans für die Zielgröße "Gleitreibung" nach einer Einlagerungszeit von 28 Tagen bei Befüllung der Glasspritzen mit Wasser bei 60°C.

Die Fig. 11 zeigt die Ergebnisse einer Pareto-Analyse des statistischen Versuchsplans für die Zielgröße "Gleitreibung" nach einer Einlagerungszeit von 28 Tagen bei Befüllung der Glasspritzen mit einem Phosphatpuffer mit einem pH-Wert von 7 bei 60°C.

Die Fig. 10 und 11 zeigen, dass aufgrund der thermischen Vorbehandlung der Glasspritzen vor der Beschichtung die Gleitreibung nach Einlagerung mit Wasser bzw. mit einem Phosphatpuffer statistisch signifikant niedriger liegt als ohne thermische Vorbehandlung. Fig. 10 und 11 zeigen weiterhin, dass der Parameter "thermische Vorbehandlung" gegenüber den Nachbehandlungsparametern Temperatur, relative Luftfeuchtigkeit sowie Nachbehandlungsdauer die stärkere Einflussgröße ist.

In einem dritten Ausführungsbeispiel ist das erfindungsgemäße Pharmapackmittel ebenfalls eine Glasspritze aus Borosilikatglas, z.B. aus FIOLAX. Das Format ist wiederum 1 ml long mit eingeklebter Nadel (27Gx1/2"). Die Spritze wird zunächst mit WFI gereinigt.

Zur erfindungsgemäßen Beschichtung wird das Perfluorpolyethersilan Daikin DSX ("Optool") mittels des Lösungsmittels HFE7200 auf eine Konzentration von 0,1% verdünnt und mit einem Magnetrührer verrührt.

Anschließend wird eine Zweistoffdüse (5mm x 90mm, Öffnungsdurchmesser 0,25mm) als "Diving Nozzle" in die Glasspritze von oben über einen Verfahrweg von 40mm eingefahren. Dabei wird die Innenseite der Glasspritze mittels eines dynamischen Sprühprozesses mit der verdünnten Lösung besprüht. Die Sprühdüse wird während dieses Prozesses über den Verfahrweg von 40mm mit einer Vorschubgeschwindigkeit von 20 mm/s aus dem Spritzenkörper heraus gefahren. Das Sprühvolumen beträgt dabei 50 µl. Der Sprühdruck liegt bei 0,5 bar, der Gasfluss bei 2,8 l/min.

In einem nächsten Schritt wird die Beschichtung auf der Spritze in einem Klimaschrank bei einer Temperatur von 50 °C und einer relativen Luftfeuchtigkeit von 50% für die Dauer von einer Stunde vernetzt.

Für die derart beschichteten Spritzen wurden die Haft- und Gleitreibungswerte gemessen. Hierfür wurde ein Teil der Spritzen mit Wasser, der andere mit einem Phosphatpuffer mit einem pH-Wert von 7 befüllt und bei einer Temperatur von 40°C in einem beschleunigten Test für eine Dauer von 28 Tagen eingelagert. Die Haft- bzw. Gleitreibungswerte wurden mittels eines FluroTec Westar RU B2-40-Stopfens gemessen.

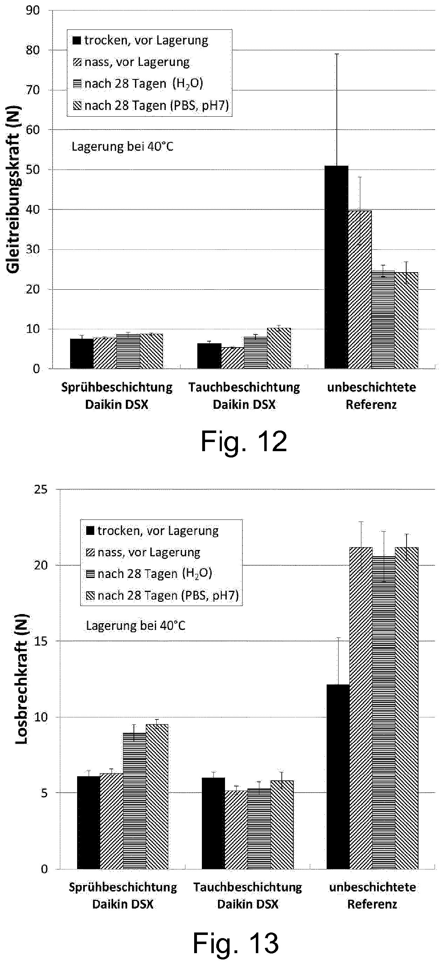

Fig. 12 zeigt die Haft- bzw. Gleitreibungswerte für unbefüllte ("trocken") und befüllte ("nass") Spritzen. Verglichen wurden hierbei Spritzen, bei denen die Beschichtung mittels eines Sprühverfahrens aufgebracht wurde, sowie Spritzen, bei denen die Beschichtung im Tauchverfahren gemäß dem ersten Ausführungsbeispiel aufgebracht wurde. Als Referenz diente wiederum eine nicht beschichtete Spritze.

Fig. 13 zeigt die Losbrechkraft für Spritzen der gleichen Art wie in Fig. 12.

Fig. 12 und 13 zeigen, dass die beschichteten Spritzen hinsichtlich der Gleitreibungs- sowie der Losbrechkraft gegenüber unbeschichteten Spritzen signifikant geringere Werte aufweisen. Dabei konnte eine signifikante Abhängigkeit dieser Abnahme der Reibungskräfte von der Art des Beschichtungsverfahrens, Sprühverfahren oder Tauchverfahren nicht festgestellt werden. Die Höhe der Abnahme ist für beide Verfahren ähnlich.

In einem vierten Ausführungsbeispiel werden Glasspritzen wie im zuvor beschriebenen dritten Ausführungsbeispiel mittels einer Zweistoffdüse beschichtet. Allerdings wurde dabei ein Perfluorpolyethersilan-haltiges Gemisch vom Typ DC2634 verwendet.

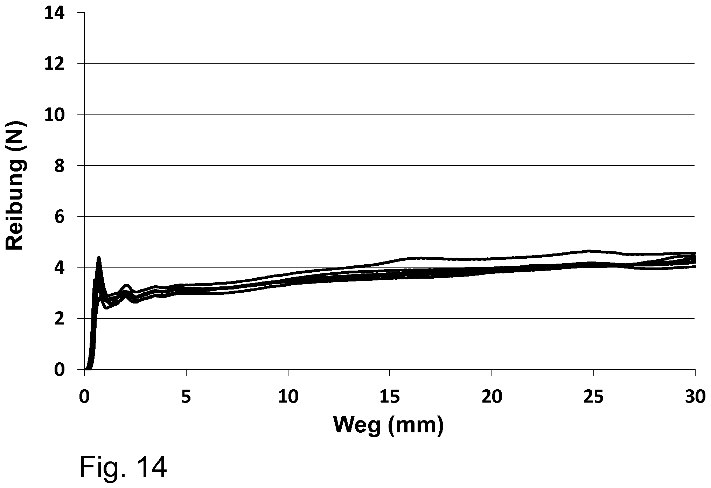

Fig. 14 zeigt den Kraft-Weg-Verlauf von mittels DC2634 beschichteter Glasspritzen. Es wurden sehr geringe Haft- bzw. Gleitreibungswerte im Bereich von 4N gemessen bei einer Geschwindigkeit von 100 mm/min.

In einem fünften Ausführungsbeispiel ist das erfindungsgemäße Pharmapackmittel ein Pharmafläschchen (Vial). Dieses wird zunächst mittels WFI gereinigt und anschließend durch folgendes Verfahren beschichtet.

Als Perfluorpolyethersilan wird Daikin DSX ("Optool") mittels des Lösungsmittels HFE7200 auf eine Konzentration von 0,1% verdünnt und mit einem Magnetrührer verrührt. Innenseitig wird das Fläschchen mit der verdünnten Lösung geflutet.

Nach einer Einwirkdauer von drei Minuten wird die überschüssige Beschichtungslösung wieder aus den Fläschchen entfernt. Anschließend werden die Fläschchen an Luft getrocknet, so dass das Lösungsmittel verdampft. In einem nächsten Schritt werden die Beschichtungen auf der Innenseite der Fläschchen in einem Klimaschrank bei einer Temperatur von 50°C und einer relativen Luftfeuchtigkeit von 50% für die Dauer einer Stunde vernetzt. Dann werden die Fläschchen mit der nachvernetzten Beschichtung mittels HFE7200 im Ultraschallbad nachgereinigt.

Die derart erfindungsgemäß beschichteten Pharmafläschchen wiesen proteinabweisende Schichten mit einem Kontaktwinkel im Bereich von 115° bis 125° auf.

In einem sechsten Ausführungsbeispiel ist das erfindungsgemäße Pharmapackmittel ebenfalls ein Pharmafläschchen (Vial). Dieses wird zunächst mittels WFI gereinigt und anschließend durch folgendes Verfahren beschichtet.

Als Perfluorpolyethersilan wird Daikin DSX ("Optool") mittels des Lösungsmittels HFE7200 auf eine Konzentration von 0,1% verdünnt und mit einem Magnetrührer verrührt.

Das Fläschchen wird in die verdünnte Lösung getaucht und dadurch auf der Außenseite beschichtet. Nach einer Haltezeit von drei Minuten wird das Fläschchen aus der Lösung gezogen und anschließend an Luft getrocknet, so dass das Lösungsmittel verdampft.

In einem nächsten Schritt werden die Fläschchen in einem Klimaschrank bei einer Temperatur von 50°C und einer relativen Luftfeuchtigkeit von 50% für die Dauer einer Stunde vernetzt. Anschließend werden die Fläschchen mit der nachvernetzten Beschichtung mittels HFE7200 im Ultraschallbad nachgereinigt.

Messungen ergaben, dass die erfindungsgemäß beschichteten Fläschchen eine wesentlich reduzierte Partikelemission, reibmindernde Eigenschaften sowie einen Kontaktwinkel im Bereich von 115° bis 120° aufwiesen.

In einem siebten Ausführungsbeispiel ist das erfindungsgemäße Pharmapackmittel ebenfalls ein Pharmafläschchen (Vial). Dieses wird zunächst mittels WFI gereinigt und anschließend durch folgendes Verfahren beschichtet.

Als Perfluorpolyethersilan wird Daikin DSX ("Optool") mittels des Lösungsmittels HFE7200 auf eine Konzentration von 0,1% verdünnt und mit einem Magnetrührer verrührt.

Eine Diving Nozzle (5m x 90mm, Öffnungsdurchmesser 0,25 mm) wird in das Fläschchen eingefahren. Die Innenseite des Fläschchens wird mit der verdünnten Lösung besprüht. Dabei wird die Sprühdüse aus dem Fläschchenkörper herausgefahren. In einem nächsten Schritt wird die Beschichtung auf dem Fläschchen in einem Klimaschrank bei einer Temperatur von 50°C und einer relativen Luftfeuchtigkeit von 50% für die Dauer einer Stunde vernetzt.

Die erfindungsgemäße Beschichtung führt für die Pharmafläschchen zu proteinabweisenden Schichten mit einem Kontaktwinkel im Bereich von 115° bis 125°.

In einem achten Ausführungsbeispiel ist das Pharmapackmittel eine Kunststoffspritze aus Cycloolefinpolymer (COC) mit dem Format 50 ml. Diese Kunststoffspritze besitzt auf ihrer Innenseite eine glasartige, d.h. Siliziumoxid-haltige Beschichtung. Die Spritze wird zunächst mit WFI gereinigt und anschließend mit folgendem Verfahren beschichtet.

Als Perfluorpolyethersilan wird Daikin DSX ("Optool") mittels des Lösungsmittels HFE7200 auf eine Konzentration von 0,1% verdünnt und mit einem Magnetrührer verrührt.

Die Spritze wird innenseitig mit der verdünnten Lösung geflutet. Nach einer Einwirkdauer von drei Minuten wird die Spritze aus der Lösung gezogen und anschließend an Luft getrocknet, so dass das Lösungsmittel verdampft.

In einem nächsten Schritt werden die Spritzen in einem Klimaschrank bei einer Temperatur von 50°C und einer relativen Luftfeuchtigkeit von 50% für die Dauer einer Stunde vernetzt. Anschließend werden die Spritzen mit der nachvernetzten Beschichtung mittels HFE7200 im Ultraschallbad nachgereinigt.

Messungen ergaben, dass die erfindungsgemäß beschichteten Spritzen eine wesentlich reduzierte Partikelemission, verbesserte Haft- bzw. Gleitreibungswerte sowie einen Kontaktwinkel im Bereich von 115° bis 120° aufwiesen.

In einem neunten Ausführungsbeispiel werden die Schichten analog zu den vorherigen Ausführungsbeispielen hergestellt jedoch wurde auf die Vorreinigung des Glassubstrats mit einem Lösungsmittel verzichtet. Es konnten dabei ähnlich gute Ergebnisse erzielt werden, so dass der Herstellprozess deutlich vereinfacht werden kann.

In einem zehnten Ausführungsbeispiel werden die Schichten analog zu den vorherigen Ausführungsbeispielen hergestellt jedoch wurde auf die Nachreinigung der Schichten mit einem Lösungsmittel verzichtet. Es konnten dabei ähnlich gute Ergebnisse erzielt werden, so dass der Herstellprozess deutlich vereinfacht werden kann.

In einem elften Ausführungsbeispiel werden die Schichten analog zu den vorherigen Ausführungsbeispielen hergestellt jedoch wurde sowohl auf einer Vorreinigung der Substrate als auch auf eine Nachreinigung der beschichteten Substrate verzichtet. Es konnten dabei ähnlich gute Ergebnisse erzielt werden, so dass der Herstellprozess deutlich vereinfacht werden kann.

In einem zwölften Ausführungsbeispiel werden die Schichten analog zu den vorherigen Ausführungsbeispielen hergestellt jedoch wurde auf die Nachvernetzung und einem feuchten Klima verzichtet. Es konnten dabei ähnlich gute Ergebnisse erzielt werden, so dass der Herstellprozess deutlich vereinfacht werden kann.

## Patentansprüche

1. Behälter mit einem Behälterkörper mit einer Außen- und einer Innenseite, wobei
- die Innenseite Siliziumoxid-haltig ist und
- die Siliziumoxid-haltige Innenseite zumindest teilweise mit einer fluorhaltigen Verbindung modifiziert ist, wobei die fluorhaltige Verbindung über zumindest eine Si-O-Si-Bindung an das Siliziumoxid des Behälterkörpers chemisch gebunden ist,
wobei die fluorhaltige Verbindung eine Alkoxysilan-Verbindung ist, wobei die Alkoxysilan-Verbindung den folgenden Aufbau hat: 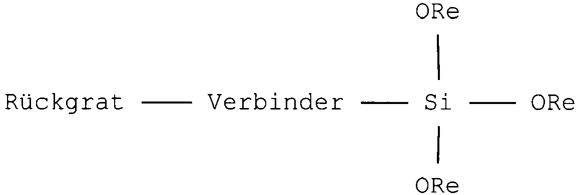 wobei ORe eine Alkoxy-Gruppe repräsentiert und wobei das Rückgrat eine fluorhaltige Einheit umfasst, wobei die Alkoxysilan-Verbindung zumindest eines der folgenden Merkmale aufweist:
- das Rückgrat enthält ein Perfluorpolyether,
- das Rückgrat umfasst zumindest eine (CF₂)₃-Kette,
- das Rückgrat umfasst mehrere (CF₂)ₓ-Einheiten, für die alle x < 8 gilt,
- das Rückgrat enthält [(CF₂)ₓO]ₙ mit 3 < n < 1000, bevorzugt 4 < n < 200, besonders bevorzugt 5 < n < 100,
- das Rückgrat enthält als weitere Verzweigungen lineare und/oder verzweigte und/oder zyklische Strukturen,
- die Alkoxysilan-Verbindung enthält wenigstens eine CF₃ Endgruppe,
und wobei der Verbinder zumindest eine hydrolysierbare Gruppe und/oder eine Aminogruppe und/oder eine Carbonsäureamid-Gruppe -OC-NH- und/oder zumindest eine weitere Silan-Gruppe und/oder eine Acrylat- oder eine Methacrylat-Gruppe enthält, wobei der Verbinder zwei oder mehrere benachbarte alkoxysilanhaltige Moleküle untereinander quer vernetzt.

2. Behälter nach Anspruch 1, wobei die mit einer fluorhaltigen Verbindung zumindest teilweise modifizierte Siliziumoxid-haltige Innenseite eine Oberflächendichte unterhalb von 2.000 Partikeln/cm² für alle Partikel mit Durchmessern ≥ 2 µm aufweist, gemessen mittels Elektronenmikroskopie, oder sich aus der Oberfläche der mit einer fluorhaltigen Verbindung modifizierten Siliziumoxid-haltigen Innenseite im Kontakt mit einer wässrigen Lösung weniger als 10.000 Partikel mit einem Durchmesser ≥ 2 µm pro ml wässriger Lösung in die wässrige Lösung übergehen, gemessen mittels eines Partikeltesters.

3. Behälter nach Anspruch 1 oder 2, wobei dessen Oberfläche frei von Ölen als Gleitmittel, insbesondere frei von Polyorganosiloxan-Verbindungen, wie beispielsweise Silikonölen ist, **gekennzeichnet durch** maximal 5 µg/cm², bevorzugt maximal 0,5 µg/cm², besonders bevorzugt 0,005 µg/cm² Masse derartiger Verbindungen, normiert auf 1 cm² modifizierter Innenoberfläche.

4. Behälter nach Anspruch 1 bis 3, wobei die modifizierte Innenseite zumindest eines der folgenden Merkmale aufweist:
- der Kontaktwinkel für Wasser beträgt mehr als 100°, bevorzugt mehr als 105°, besonders bevorzugt mehr als 110°,
- der dynamische Kontaktwinkel beträgt beim Eintauchen mehr als 110° und im Rückzug mehr als 90°, vorzugsweise beim Eintauchen mehr als 115° und im Rückzug mehr als 105°,
- der Abrollwinkel liegt im Bereich von 1° bis 30°, bevorzugt im Bereich von 5° bis 20°, gemessen für ein Tröpfchen von 60 µl.

5. Verfahren zur Herstellung eines Behälters nach Anspruch 1 bis 4 mit geringer Partikelemission mit den Schritten:
a) Bereitstellen eines Behälterkörpers mit Außen- und einer Innenseite, wobei die Innenseite Siliziumoxid-haltig ist,
b) Aufbringen eines Gemisches aus einer fluororganischen Verbindung gelöst in einem Lösungsmittel auf zumindest einem Teil der Innenseite des Behälterkörpers, wobei die fluorhaltige Verbindung eine Alkoxysilan-Verbindung ist, wobei die Alkoxysilan-Verbindung den folgenden Aufbau hat: 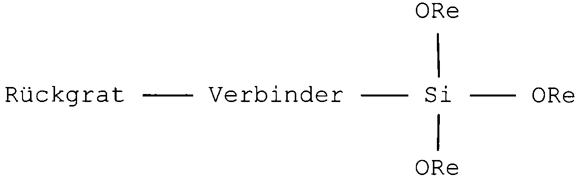
wobei der Verbinder zumindest eine hydrolysierbare Gruppe und/oder eine Aminogruppe und/oder eine Carbonsäureamid-Gruppe -OC-NH- und/oder zumindest eine weitere Silan-Gruppe und/oder eine Acrylat- oder eine Methacrylat-Gruppe und wobei ORe eine Alkoxy-Gruppe repräsentiert und wobei das Rückgrat eine fluorhaltige Einheit umfasst.c) Trocknung und Vernetzung der fluorhaltigen Verbindung mit Siliziumoxid an der Innenseite des Behälterkörpers mittels einer Kondensationsreaktion,
sowie
Quervernetzung der fluorhaltigen Verbindungen untereinander mittels des Verbinders.

6. Verfahren nach Anspruch 5, wobei in Schritt b) ein fluorhaltiges Lösungsmittel verwendet wird, das zumindest eine der folgenden Verbindungen enthält:
- Ethoxynonafluorbutan,
- Methoxynonafluorbutan,
- Perfluorhexan,
- Hydrofluorether.

7. Verfahren nach Anspruch 5 oder 6, wobei die Vernetzung bei einer Temperatur oberhalb von 30°C oder einer relativen Luftfeuchtigkeit im Bereich 10% bis 95%, besonders bevorzugt im Bereich 30% bis 70% erfolgt.

8. Verfahren nach Anspruch 5 bis 7, wobei in Schritt a) die Innenseite der Behälteroberfläche zumindest teilweise vorbehandelt wird, wobei das Vorbehandeln in zumindest einem der folgenden Schritte durchgeführt wird:
- Thermisches Vorbehandeln bei einer Temperatur oberhalb 350 °C, bevorzugt oberhalb 400 °C, besonders bevorzugt oberhalb 500 °C,
- Waschen mit sterilem, partikelarmen Wasser,
- nass-chemisches Vorbehandeln mit einer sauren oder einer basischen Lösung,
- Reinigen gemäß einem der beiden vorstehenden Schritte unter Anwendung von Ultraschall mit einer Frequenz im Bereich 20 kHz bis 2,5 MHz, bevorzugt mit einer Frequenz im Bereich 100 kHz bis 2 MHz,
- Trocknen, vorzugsweise mittels Einblasen von Luft oder in einem Durchlaufofen.

9. Verfahren nach Anspruch 5 bis 8, wobei zwischen den Schritten a) und b) in einem Schritt a1) eine Zwischenschicht auf zumindest einem Teilbereich der Innenseite des Behälterkörpers aufgebracht wird,
wobei die Zwischenschicht mindestens eines der folgenden Merkmale aufweist:
- Die Zwischenschicht fungiert als Haftvermittler-Schicht,
- die Zwischenschicht enthält Siliziumoxid,
- die Zwischenschicht ist Sol Gel-basiert,
- die Zwischenschicht enthält wenigstens eine unter- oder überstöchiometrische Oxidverbindung,
- die Zwischenschicht ist mit weiteren Verbindungen dotiert,
- die Zwischenschicht umfasst ein Mischoxid, bevorzugt ein dotiertes Siliziumoxid, besonders bevorzugt ein mit einem Oxid der Elemente Al, Mg, P, Ce, Zr, Ti, Ba, Sr, Nb, B oder mit Magnesiumfluorid dotiertes Siliziumoxid.

10. Verfahren nach Anspruch 5 bis 9, wobei die Innenseite der Behälteroberfläche nach Schritt c) zumindest teilweise nachbehandelt wird, wobei das Nachbehandeln in zumindest einem der folgenden Schritte durchgeführt wird:
- Trocknen unter Umgebungsbedingungen oder in einem Ofen,
- Nachreinigen mittels Ultraschallbad und/oder unter Verwendung eines Lösungsmittels und/oder unter Verwendung eines fluorhaltigen Lösungsmittels und/oder unter Verwendung des gleichen Lösungsmittels wie in Schritt b) und/oder unter Verwendung von Wasser, bevorzugt von Water for Injection (WFI).

11. Verwendung des Behälters nach Anspruch 1 als Spritzen- oder als Karpulensystem oder als medizinischer Vorrichtung mit geringer Partikelemission oder mit reibkontrollierter Oberfläche zur Aufbewahrung von pharmazeutischen Wirkstofflösungen, insbesondere von Protein-basierten pharmazeutischen Wirkstoffformulierungen.

## Claims

1. A container comprising a container body with an outer surface and an inner surface, wherein
- the inner surface contains silicon oxide; and
- the silicon oxide containing inner surface is at least partially modified with a fluorine containing compound, wherein the fluorine containing compound is chemically bonded to the silicon oxide of the container body via at least one Si-O-Si bond;
wherein the fluorine containing compound is an alkoxysilane compound, said alkoxysilane compound having a structure as follows: 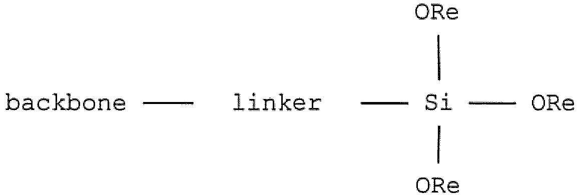 wherein ORe represents an alkoxy group, and wherein the backbone comprises a fluorine containing entity;
wherein the alkoxysilane compound has at least one of the following features:
- the backbone comprises a perfluoropolyether;
- the backbone comprises at least one (CF₂)₃ chain;
- the backbone comprises a plurality of (CF₂)ₓ entities, for all of which x < 8 is met;
- the backbone comprises [(CF₂)ₓO]ₙ, with 3 < n < 1000, preferably 4 < n < 200, more preferably 5 < n < 100;
- the backbone comprises further branches in the form of linear and/or branched and/or cyclic structures;
- the alkoxysilane compound comprises at least one CF₃ end group; and
- the linker comprises at least one hydrolyzable group, and/or an amino group, and/or a carboxamide group -OC-NH-, and/or at least one further silane group, and/or an acrylate or methacrylate group; wherein the linker crosslinks two or more adjacent alkoxysilane molecules to each other.

2. The container according to claim 1, wherein the silicon oxide containing inner surface that is at least partially modified with a fluorine containing compound has a surface density of less than 2,000 particles/cm² for any particles of a diameter of ≥ 2 pm, as measured by electron microscopy, or wherein in contact with an aqueous solution less than 10,000 particles of a diameter of ≥ 2 µm, as measured with a particle tester, are released per ml of the aqueous solution from the silicon oxide containing inner surface modified with a fluorine containing compound into the aqueous solution.

3. The container according to claim 1 or 2, wherein the surface thereof is free of lubricating oils, in particular free of polyorganosiloxane compounds such as silicone oils, **characterized by** not more than 5 µg/cm², preferably not more than 0.5 µg/cm², more preferably 0.005 g/cm² mass of such compounds normalized to 1 cm² of the modified inner surface.

4. The container according to claim 1 to 3, wherein the modified inner surface has at least one of the following features:
- the contact angle to water is greater than 100°, preferably greater than 105°, more preferably greater than 110°;
- the dynamic contact angle is greater than 110° upon immersion and greater than 90° upon retraction, preferably greater than 115° upon immersion and greater than 105° upon retraction;
- the roll-off angle is in a range from 1° to 30°, preferably in a range from 5° to 20°, as measured for a droplet of 60 µl.

5. A method for producing a container according to claim 1 to 4 with low particulate emission, comprising the steps of:
a) providing a container body having an outer surface and an inner surface, wherein the inner surface contains silicon oxide;
b) applying a mixture of an organic fluorine compound dissolved in a solvent to at least a portion of the inner surface of the container body, wherein the fluorine containing compound is an alkoxysilane compound, wherein said alkoxysilane compound has a structure as follows: 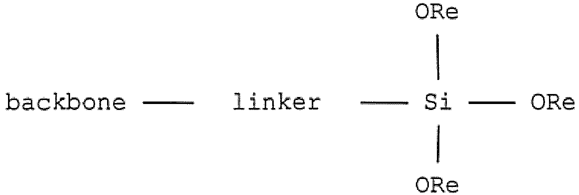 wherein the linker comprises at least one hydrolyzable group, and/or an amino group, and/or a carboxamide group -OC-NH-, and/or at least one further silane group, and/or an acrylate or methacrylate group; and
wherein ORe represents an alkoxy group, and wherein the backbone comprises a fluorine containing entity;
c) drying the fluorine containing compound and crosslinking it with silicon oxide at the inner surface of the container body by a condensation reaction; and crosslinking the fluorine containing compounds with each other via the linker.

6. The method according to claim 5, wherein a fluorine containing solvent is used in step b), which includes at least one of the following compounds:
- Ethoxynonafluorobutane;
- Methoxynonafluorobutane;
- Perfluorohexane;
- Hydrofluoroether.

7. The method according to claim 5 or 6, wherein the crosslinking is accomplished at a temperature above 30 °C or at a relative humidity in a range from 10 % to 95 %, more preferably in a range from 30 % to 70 %.

8. The method according to claim 5 to 7, wherein in step a) the inner surface of the container is pretreated, at least partially, wherein the pretreating is performed by at least one of the steps of:
- thermal pretreatment at a temperature above 350 °C, preferably above 400 °C, more preferably above 500 °C;
- washing with sterile, low particulate water;
- wet-chemical pretreatment using an acidic or an alkaline solution;
- cleaning according to any of the two preceding steps using ultrasound at a frequency in a range from 20 kHz to 2.5 MHz, preferably at a frequency in a range from 100 kHz to 2 MHz;
- drying, preferably by blowing in air, or in a continuous furnace.

9. The method according to claim 5 to 8, wherein in a step a1) between steps a) and b) an intermediate layer is applied upon at least a portion of the inner surface of the container body, wherein said intermediate layer has at least one of the following features:
- the intermediate layer functions as an adhesion promoting layer;
- the intermediate layer contains silicon oxide;
- the intermediate layer is a sol-gel-based layer;
- the intermediate layer comprises at least one under-stoichiometric or over-stoichiometric oxide compound;
- the intermediate layer is doped with further compounds;
- the intermediate layer comprises a mixed oxide, preferably a doped silicon oxide, more preferably a silicon oxide doped with an oxide of elements Al, Mg, P, Ce, Zr, Ti, Ba, Sr, Nb, B, or with magnesium fluoride.

10. The method according to claim 5 to 9, wherein after step c) the inner surface of the container is subjected to a posttreatment, at least partially, wherein the posttreatment is performed by at least one of the following steps:
- drying under ambient conditions or in a furnace;
- post-cleaning by means of an ultrasonic bath, and/or using a solvent, and/or using a fluorine containing solvent, and/or using the same solvent as in step b), and/or using water, preferably water for injection (WFI) .

11. Use of the container according to claim 1 as a syringe system or cartridge system or as a medical device with low particulate emission or with friction controlled surface for storing pharmaceutical drug solutions, in particular protein-based pharmaceutical drug formulations.

## Revendications

1. Récipient doté d'un corps de récipient ayant une face extérieure et une face intérieure, dans lequel :
- la face intérieure comprend de l'oxyde de silicium et
- la face intérieure contenant de l'oxyde de silicium est modifiée au moins partiellement avec un composé fluoré, le composé fluoré étant lié chimiquement à l'oxyde de silicium du corps de récipient par le biais d'au moins une liaison Si-O-Si,
dans lequel le composé fluoré est un composé alkoxysilane, qui possède la structure suivante : 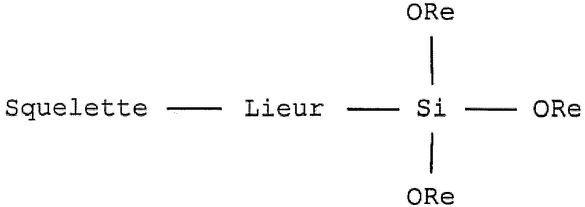 où ORe représente un groupe alkoxy et où le squelette comprend une unité fluorée,
dans lequel le composé alkoxysilane comprend au moins l'une des caractéristiques suivantes :
- le squelette contient un perfluoropolyéther,
- le squelette comprend au moins une chaîne (CF₂)₃,
- le squelette comprend plusieurs unités (CF₂)ₓ, où tous les x < 8,
- le squelette contient [(CF₂)ₓO]ₙ avec 3 < n < 1 000, de préférence 4 < n < 200, de manière particulièrement préférée 5 < n < 100,
- le squelette contient comme autres ramifications des structures linéaires et/ou ramifiées et/ou cycliques,
- le composé alkoxysilane contient au moins un groupe terminal CF₃,
et dans lequel le lieur contient au moins un groupe hydrolysable et/ou un groupe amino et/ou un groupe amide d'acide carboxylique -OC-NH- et/ou au moins un autre groupe silane et/ou un groupe acrylate ou méthacrylate, le lieur réticulant transversalement deux molécules ou plus de deux molécules adjacentes contenant de l'alkoxysilane.

2. Récipient selon la revendication 1, dans lequel la face intérieure contenant de l'oxyde de silicium, partiellement modifiée par un composé fluoré, présente une densité surfacique inférieure à 2 000 particules/cm² pour toutes les particules ayant des diamètres ≥ 2 pm, mesurée par microscopie électronique, ou moins de 10 000 particules ayant un diamètre ≥ 2 pm par ml de solution aqueuse se transforment en solution aqueuse depuis la surface de la face intérieure contenant de l'oxyde de silicium, modifiée par un composé fluoré, au contact d'une solution aqueuse, mesurées par un testeur de particules.

3. Récipient selon la revendication 1 ou 2, dans lequel sa surface est exempte d'huiles comme agent de glissement, en particulier exempte de composés polyorganosiloxane, comme par exemple les huiles siliconées, **caractérisé par** au maximum 5 µg/cm², de préférence au maximum 0,5 µg/cm², de manière particulièrement préférée 0,005 µg/cm² de masse de composés de ce type, normalisée par rapport à 1 cm² de surface intérieure modifiée.

4. Récipient selon les revendications 1 à 3, dans lequel la face intérieure modifiée comporte au moins l'une des caractéristiques suivantes :
- l'angle de contact pour l'eau atteint plus de 100°, de préférence plus de 105°, de manière particulièrement préférée plus de 110°,
- l'angle de contact dynamique atteint lors de l'immersion plus de 110° et au retrait plus de 90°, de préférence lors de l'immersion plus de 115° et au retrait plus de 105°,
- l'angle de mouillage est compris dans la plage de 1° à 30°, de préférence dans la plage de 5° à 20°, mesurée pour une gouttelette de 60 µl.

5. Procédé de fabrication d'un récipient selon les revendications 1 à 4 avec moins d'émission de particules, comportant les étapes de :
a) mise à disposition d'un corps de récipient doté d'une face extérieure et d'une face intérieure, la face intérieure contenant de l'oxyde de silicium,
b) dépôt d'un mélange d'un composé organofluoré dissous dans un solvant sur au moins une partie de la face intérieure du corps de récipient, le composé fluoré étant un composé alkoxysilane, le composé alkoxysilane possédant la structure suivante : 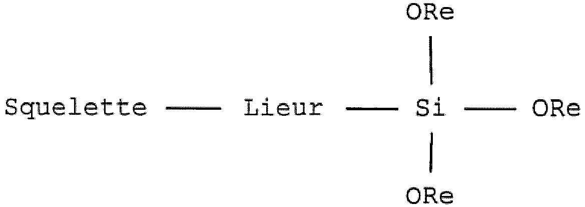 où le lieur contient au moins un groupe hydrolysable et/ou un groupe amino et/ou un groupe amide d'acide carboxylique -OC-NH- et/ou au moins un autre groupe silane et/ou un groupe acrylate ou méthacrylate, et où ORe représente un groupe alkoxy et où le squelette comporte une unité fluorée,
c) séchage et réticulation du composé fluoré avec l'oxyde de silicium sur la face intérieure du corps de récipient au moyen d'une réaction de condensation, et réticulation transversale des composés fluorés entre eux au moyen du lieur.

6. Procédé selon la revendication 5, dans lequel, à l'étape b), un solvant fluoré est utilisé, qui contient au moins l'un des composés suivants :
- éthoxynonafluorobutane,
- méthoxynonafluorobutane,
- perfluorohexane,
- hydrofluoréther.

7. Procédé selon la revendication 5 ou 6, dans lequel la réticulation s'effectue à une température supérieure à 30°C ou à une humidité de l'air relative comprise dans la plage de 10 % à 95 %, de manière particulièrement préférée dans la plage de 30 % à 70 %.

8. Procédé selon les revendications 5 à 7, dans lequel, à l'étape a), la face intérieure de la surface du récipient est prétraitée au moins partiellement, le prétraitement étant effectué au cours d'au moins l'une des étapes suivantes :
- prétraitement thermique à une température supérieure à 350°C, de préférence supérieure à 400°C, de manière particulièrement préférée supérieure à 500°C,
- lavage avec de l'eau stérile, pauvre en particules,
- prétraitement chimique par voie humide avec une solution acide ou basique,
- purification selon l'une des deux étapes précédentes en appliquant des ultrasons à une fréquence dans la plage de 20 kHz à 2,5 MHz, de préférence à une fréquence dans la plage de 100 kHz à 2 MHz,
- séchage de préférence au moyen de l'insufflation d'air ou dans un four continu.

9. Procédé selon les revendications 5 à 8, dans lequel, entre les étapes a) et b), au cours d'une étape a1), une couche intermédiaire est déposée sur au moins une zone partielle de la face intérieure du corps de récipient, la couche intermédiaire présentant au moins l'une des caractéristiques suivantes :
- la couche intermédiaire fait office de couche d'adhérence,
- la couche intermédiaire contient de l'oxyde de silicium,
- la couche intermédiaire est à base de sol-gel,
- la couche intermédiaire comprend au moins un composé d'oxyde sous- ou sur-stoechiométrique,
- la couche intermédiaire est dopée avec d'autres composés,
- la couche intermédiaire comprend un oxyde mixte, de préférence un oxyde de silicium dopé, de manière particulièrement préférée un oxyde de silicium dopé avec un oxyde des éléments Al, Mg, P, Ce, Zr, Ti, Ba, Sr, Nb, B ou du fluorure de magnésium.

10. Procédé selon les revendications 5 à 9, dans lequel la face intérieure de la surface de récipient est traitée ultérieurement au moins partiellement après l'étape c), le traitement ultérieur étant effectué au cours d'au moins l'une des étapes suivantes :
- séchage dans des conditions environnementales ou dans un four,
- purification ultérieure au moyen d'un bain d'ultrasons et/ou en utilisant un solvant et/ou en utilisant un solvant fluoré et/ou en utilisant le même solvant que celui de l'étape b) et/ou en utilisant de l'eau, de préférence de l'eau pour injection (WFI).

11. Utilisation du récipient selon la revendication 1 comme système de seringues ou d'ampoules ou comme dispositif médical à faible émission de particules ou ayant une surface à friction contrôlée pour la conservation des solutions de substances actives pharmaceutiques, en particulier des formulations de substances actives pharmaceutiques à base de protéines.
